(19) Europäisches Patentamt
European Patent Office
Office européen des brevets

(11) EP 2 024 088 B2

(12) # NEUE EUROPÄISCHE PATENTSCHRIFT

Nach dem Einspruchsverfahren

(45) Veröffentlichungstag und Bekanntmachung des Hinweises auf die Entscheidung über den Einspruch:
**01.08.2018 Patentblatt 2018/31**

(45) Hinweis auf die Patenterteilung:
**04.07.2012 Patentblatt 2012/27**

(21) Anmeldenummer: **07724525.6**

(22) Anmeldetag: **24.04.2007**

(51) Int Cl.:
***B01J 31/02*** $^{(2006.01)}$ ***B01J 37/02*** $^{(2006.01)}$

(86) Internationale Anmeldenummer:
**PCT/EP2007/003595**

(87) Internationale Veröffentlichungsnummer:
**WO 2007/124896 (08.11.2007 Gazette 2007/45)**

(54) **MIT EINER IONISCHEN FLÜSSIGKEIT BESCHICHTETER PORÖSER HETEROGENER KATALYSATOR**

POROUS HETEROGENEOUS CATALYST COATED WITH AN IONIC FLUID

CATALYSEUR HÉTÉROGÈNE POREUX RECOUVERT D'UN LIQUIDE IONIQUE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **26.04.2006 DE 102006019460**

(43) Veröffentlichungstag der Anmeldung:
**18.02.2009 Patentblatt 2009/08**

(73) Patentinhaber: **Clariant Produkte (Deutschland) GmbH**
**65929 Frankfurt am Main (DE)**

(72) Erfinder:
- **JESS, Andreas**
  **95447 Bayreuth (DE)**
- **KORTH, Wolfgang**
  **95447 Bayreuth (DE)**
- **ETZOLD, Bastian**
  **91054 Buckenhof (DE)**

(74) Vertreter: **Stolmár & Partner**
**Patentanwälte PartG mbB**
**Blumenstraße 17**
**80331 München (DE)**

(56) Entgegenhaltungen:
**DE-A1- 10 361 071 US-A1- 2004 267 034**

- **MIKKOLA, JYRI-PEKKA ET AL: "Supported ionic liquids catalysts for fine chemicals: citral hydrogenation" GREEN CHEMISTRY ( 2006 ), 8(2), 197-205 CODEN: GRCHFJ; ISSN: 1463-9262, 12. Oktober 2005 (2005-10-12), XP002444151**
- **DECASTRO C ET AL: "Immobilised Ionic Liquids as Lewis Acid Catalysts for the Alkylation of Aromatic Compounds with Dodecene" JOURNAL OF CATALYSIS, ACADEMIC PRESS, DULUTH, MN, US, Bd. 196, Nr. 1, 15. November 2000 (2000-11-15), Seiten 86-94, XP004465156 ISSN: 0021-9517**
- **RIISAGER, ANDERS ET AL: "Supported ionic liquid phase (SILP) catalysis. An innovative concept for homogeneous catalysis in continuous fixed-bed reactors" EUROPEAN JOURNAL OF INORGANIC CHEMISTRY ( 2006 ), (4), 695-706 CODEN: EJICFO; ISSN: 1434-1948, 1. Februar 2006 (2006-02-01), XP002444152**
- **RIISAGER A. ET AL: 'Stability and kinetic studies of supported ionic liquid phase catalysts for hydroformylation of propene' INDUSTRIAL ENGINEERING CHEMISTRY RESEARCH Bd. 44, 2005, Seiten 9853 - 9859**
- **HUANG J. ET AL: 'Pd nanoparticles immobilized on molecular sieves by ionic liquids: Heterogeneous catalysts for solvent-free hydrogenation' ANGEWANDTE CHEMIE Bd. 43, 2004, Seiten 1397 - 1399**
- **XU D-Q. ET AL: 'Hydrogenation in ionic liquids:: An alternative methodology toward highly selective catalysis of halonitrobenzenes to corresponding haloanilines' JOURNAL OF MOLECULAR CATALYSIS A: CHEMICAL Bd. 235, 2005, Seiten 137 - 142**



Printed by Jouve, 75001 PARIS (FR)

## Beschreibung

Die vorliegende Erfindung betrifft einen porösen heterogenen Übergangsmetallkatalysator.

Selektive Hydrierungsreaktionen sind beispielsweise bei der Verarbeitung von durch Erdölraffination gewonnenen Kohlenstoffschnitten zu synthetisch wertvollen Zwischenprodukten von großer Bedeutung, wie beispielsweise bei der selektiven Hydrierung von aromatischen Verbindungen zu Cycloolefinen, bei der Hydrierung von Acetylen zu Ethylen, bei der Hydrierung von Butadien zu 1-Buten und/oder 2-Buten und bei der Hydrierung von Phenylacetylen zu Styrol.

Die bei selektiven Hydrierungsreaktionen eingesetzten Katalysatoren sind häufig geträgerte Metallkatalysatoren, beispielsweise geträgerte Nickel-, Palladium- oder Platinkatalysatoren. Diese Katalysatoren beschleunigen die gewünschte Hydrierungsreaktion und besitzen bezüglich dieser zwar auch eine gewisse Selektivität, der Anteil an unerwünschten Nebenprodukten ist insbesondere bei hohen Umsatzraten jedoch in der Regel immer noch verhältnismäßig hoch.

In dem Journal "Green Chem, 2006, 8, 197-205" ist ein heterogener katalysator offenbart, bei dem Palladium in einer ionischen Flüssigkeit gelöst ist.

Aufgabe der vorliegenden Erfindung ist es daher, einen Katalysator bereitzustellen, der mit einer verhältnismäßig hohen Selektivität die Hydrierung einzelner ungesättigter Bindungen mehrfach ungesättigter Verbindungen katalysiert.

Diese Aufgabe wird gelöst gemäß den Merkmalen in Anspruch 1. Vorteilhafte Ausgestaltungen befinden sich in den Unteransprüchen.

Der erfindungsgemäße Katalysator weist bei der Hydrierung einzelner ungesättigter Bindungen mehrfach ungesättigter Verbindungen eine verhältnismäßig hohe Selektivität auf bei relativ hoher Umsatzrate.

Der erfindungsgemäße Katalysator kann unter Verwendung leicht zugänglicher Einsatzstoffe verfahrenstechnisch einfach und damit kostengünstig hergestellt werden.

Der erfindungsgemäße Katalysator hat ferner den Vorteil, dass er zur Katalyse von Reaktionen in gasförmiger oder flüssiger Phase eingesetzt werden kann. Damit die hohe Selektivität des erfindungsgemäßen Katalysators möglichst lange gewährleistet ist, ist der Katalysator vorzugsweise aber nur solchen Reaktionsbedingungen zu unterwerfen, welche im Wesentlichen kein Ablösen der IL-Beschichtung bewirken.

Wie bereits vorstehend ausgeführt, ist der erfindungsgemäße Katalysator ein poröser heterogener Übergangsmetallkatalysator, dessen innere Oberfläche mit einer ionischen Flüssigkeit beschichtet ist. Dabei ist der poröse heterogene Übergangsmetallkatalysator selbst ein präformierter Katalysator, d.h., dass der erfindungsgemäße Katalysator hergestellt wird, indem ein vollständig ausgebildeter poröser heterogener Katalysator eingesetzt und dieser mit der ionischen Flüssigkeit beschichtet wird.

Geeignete Katalysatoren enthalten Metalle, die ausgewählt sind aus der Gruppe bestehend aus Nickel, Kobalt, Kupfer, Eisen, Ruthenium, Rhodium, Iridium, Palladium und Platin. Die heterogenen Katalysatoren können dotiert oder undotiert sein. Geeignete Dotiermetalle können ausgewählt werden aus den Elementen der Gruppen 3 bis 12 des Periodensystems der Elemente gemäß IUPAC-Nomenklatur (Handbook of Chemistry and Physics, 80. Ausgabe, 1999-2000).

Das katalytisch aktive Metall ist in einer Menge von vorzugsweise 0,1 bis 60 Gew.-%, besonders bevorzugt 1 bis 50 Gew.-%, insbesondere 2 bis 10 Gew.-% vorhanden bezogen auf das Gesamtgewicht des erfindungsgemäßen Katalysators.

Ionische Flüssigkeiten sind nach der Definition von Wasserscheid und Keim in "Angewandte Chemie" 2000, 112, Seiten 3926-3945 bei relativ niedriger Temperatur schmelzende Salze. Ionische Flüssigkeiten sind daher bereits bei relativ niedrigen Temperaturen flüssig. Darüber hinaus sind sie im Allgemeinen nicht brennbar und haben keinen messbaren Dampfdruck.

Im Rahmen der vorliegenden Erfindung werden unter dem Begriff ionische Flüssigkeit Salze verstanden, die einen Schmelzpunkt oder Schmelzbereich aufweisen, der unterhalb von 200 °C liegt, vorzugsweise unterhalb von 150 °C und besonders bevorzugt unterhalb von 100 °C.

Ferner sind bevorzugt die ionischen Flüssigkeiten solche, die eine Molmasse von vorzugsweise höchstens 1.000 g/mol aufweisen, besonders bevorzugt höchstens 500 g/mol.

Ferner sind bevorzugte ionische Flüssigkeiten solche, deren Kationen organischer Natur sind und deren Anionen organischer oder anorganischer Natur sind.

Ionische Flüssigkeiten werden aus positiven und negativen Ionen gebildet, sind jedoch insgesamt ladungsneutral. Die positiven wie auch die negativen Ionen sind überwiegend einwertig, möglich sind jedoch auch multivalente Anionen und/oder Kationen, die bis zu fünf, vorzugsweise bis zu vier, besonders bevorzugt bis zu drei und besonders bevorzugt bis zu zwei elektrische Ladungen aufweisen. Dabei können die Ladungen innerhalb des jeweiligen Ions entweder lokalisiert oder delokalisiert sein.

Die vorliegende Erfindung ist nicht auf Katalysatoren beschränkt, deren innere Oberfläche mit einer bestimmten

ionischen Flüssigkeit beschichtet ist; es können alle geeigneten ionischen Flüssigkeiten verwendet sein, worunter auch Gemische verschiedener ionischer Flüssigkeiten verstanden werden.

Gemäß einer bevorzugten Ausführungsform der Erfindung ist der erfindungsgemäße Katalysator als Pulver oder als Formkörper ausgebildet.

Als Pulver kann der erfindungsgemäße Katalysator mit hohen Ausbeuten und Selektivitäten in Suspensionsverfahren eingesetzt werden. Typische Partikelgrößen solcher Pulver liegen bei 10 bis 250 Mikrometer, es können aber auch Partikel deutlich kleiner als 1 Mikrometer verwendet werden, etwa beim Einsatz von Ruß als Katalysatorträger.

Formkörper kommen beispielsweise bei im Festbett betriebenen Prozessen bevorzugt zum Einsatz. Bevorzugte Formkörper sind Kugeln, Kegel, Stränge, Hohlstränge, Sternstränge, Vollzylinder, Hohlzylinder, Tabletten, Triloben, Splitt, etc. mit charakteristischen Durchmessern von 0,5 bis 5 mm oder auch Monolithe und ähnliche strukturierte Packungen (vgl. Ullmann's Encyclopedia, Sixth Edition, 2000 Electronic Release, Chapter Fixed-Bed Reactors, Par. 2: Catalyst Forms for Fixed-Bed Reactors).

Es wurde festgestellt, dass mittels Beschichten eines heterogenen porösen Katalysators mit einer ionischen Flüssigkeit die Aktivität des Katalysators so stark abgesenkt werden kann, dass auch Formkörper mit einem Durchmesser von bis zu 2cm eingesetzt werden können, ohne nennenswerte Verluste hinsichtlich der Produktselektivität hinnehmen zu müssen. Bevorzugte Formkörper weisen daher einen Durchmesser oder Abmessungen von 1 mm bis 2 cm auf, vorzugsweise von 2 mm bis 1,8 cm, bevorzugt von 4 mm bis 1,5 cm und mehr bevorzugt von 6 mm bis 1,2 cm.

Es handelt sich bevorzugt um einen geträgerten Edelmetallkatalysator.

Es kann bevorzugt sein, dass die BET-Oberfläche des Katalysators ohne die IL-Beschichtung 10 bis 300 $m^2/g$ beträgt, vorzugsweise 15 bis 80 $m^2/g$, besonders bevorzugt 20 bis 50 $m^2/g$. Die BET-Oberfläche wird nach der Einpunkt-Methode durch Adsorption von Stickstoff nach DIN 66132 bestimmt.

Ferner kann es bevorzugt sein, dass die BET-Oberfläche des Katalysators mit der IL-Beschichtung 8 bis 240 $m^2/g$ beträgt, vorzugsweise 12 bis 64 $m^2/g$, besonders bevorzugt 16 bis 40 $m^2/g$.

Darüber hinaus kann es bevorzugt sein, dass das integrale Porenvolumen des Katalysators (bestimmt gemäß DIN 66133 (Hg-Porosimetrie)) ohne die IL-Beschichtung größer als 100 $mm^3/g$ ist, vorzugsweise größer als 180 $mm^3/g$.

Es kann auch bevorzugt sein, dass das integrale Porenvolumen des Katalysators mit der IL-Beschichtung größer als 80 $mm^3/g$ ist, vorzugsweise größer als 144 $mm^3/g$.

Entsprechend einer bevorzugten Ausführungsform des erfindungsgemäßen Katalysators sind maximal 10 % des Porenvolumens des Katalysators ohne die IL-Beschichtung von Poren mit einem Radius von kleiner 10 nm gebildet, vorzugsweise maximal 8 %, bevorzugt maximal 6 % und besonders bevorzugt maximal 5 %.

Gemäß einer weiteren bevorzugten Ausführungsform des erfindungsgemäßen Katalysators sind maximal 10 % des Porenvolumens des Katalysators ohne die IL-Beschichtung von Poren mit einem Radius von größer als 500 nm gebildet, vorzugsweise maximal 8 %, bevorzugt maximal 6 % und besonders bevorzugt maximal 5 %.

Bei einer weiteren bevorzugten Ausführungsform des erfindungsgemäßen Katalysators ist vorgesehen, dass der mittlere Porendurchmesser des Katalysators ohne die IL-Beschichtung 10 bis 100 nm beträgt.

Darüber hinaus kann gemäß einer bevorzugten Weiterbildung des erfindungsgemäßen Katalysators der mittlere Porendurchmesser des Katalysators mit der IL-Beschichtung 5 bis 100 nm betragen.

Grundsätzlich kann im Rahmen der vorliegenden Erfindung der erfindungsgemäße Katalysator mit einer beliebigen ionischen Flüssigkeit beschichtet sein und entsprechend das Kation beliebiger Natur sein. Bevorzugt als Kation sind allgemein beispielsweise Ammonium- oder Phosphoniumionen oder solche Kationen, die mindestens einen fünf- oder sechsgliedrigen Heterocyclus enthalten, der mindestens ein Phosphor- oder ein Stickstoffatom sowie gegebenenfalls ein Sauerstoff- oder Schwefelatom aufweist. Besonders bevorzugt sind Kationen, die mindestens einen fünf- oder sechsgliedrigen Heterocyclus enthalten, der ein, zwei oder drei Stickstoffatome und ein Schwefel- oder ein Sauerstoffatom aufweist. Ganz besonders bevorzugt sind Kationen, die mindestens einen fünf- oder sechsgliedrigen Heterocyclus enthalten, der ein oder zwei Stickstoffatome aufweist.

Es kann bevorzugt sein, dass das Kation der ionischen Flüssigkeit ausgewählt ist aus Verbindungen der nachstehenden allgemeinen Formeln IL-1 bis IL-23:

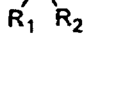

(IL-1)

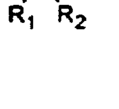

(IL-2)

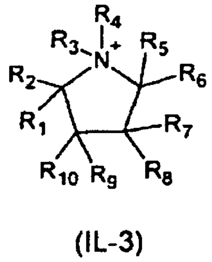

(IL-3)

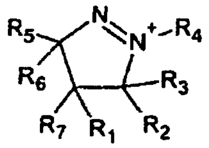

(IL-4)

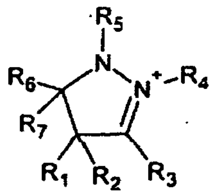

(IL-5)

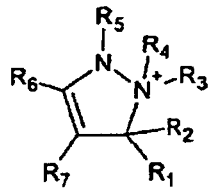

(IL-6)

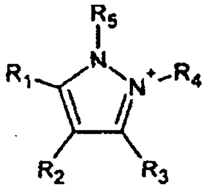

(IL-7)

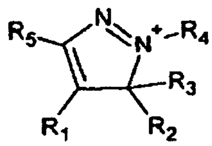

(IL-8)

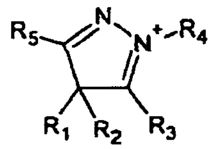

(IL-9)

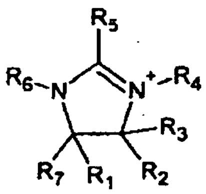

(IL-10)

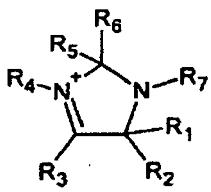

(IL-11)

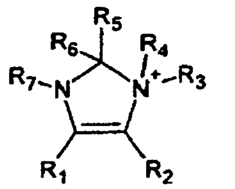

(IL-12)

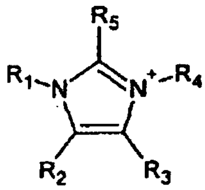

(IL-13)

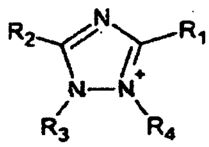

(IL-14)

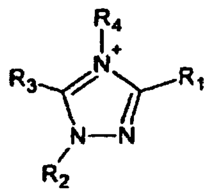

(IL-15)

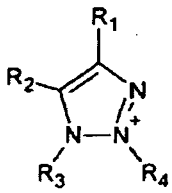

(IL-16)

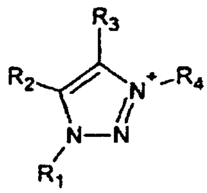

(IL-17)

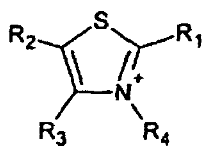

(IL-18)

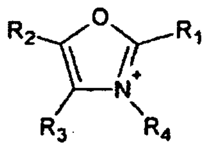

**(IL-19)**

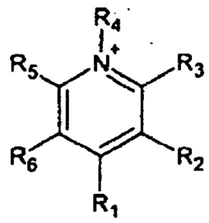

**(IL-20)**

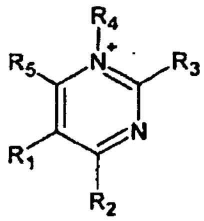

**(IL-21)**

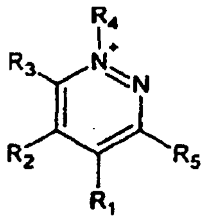

**(IL-22)**

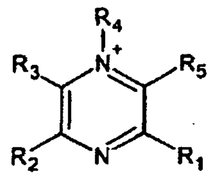

**(IL-23)**

worin die Reste $R_1$, $R_2$, $R_3$, $R_4$, $R_5$, $R_6$, $R_7$, $R_8$, $R_9$ und $R_{10}$ jeweils unabhängig voneinander Reste sein können ausgewählt aus der Gruppe bestehend aus Wasserstoff, funktionelle Gruppen, Aryl, Alkyl, Aryloxy, Alkyloxy, Halogen, Heteroatom und/oder Heterocyclus substituiertes $C_1$-$C_{18}$-Alkyl, durch ein oder mehrere nicht benachbarte Sauerstoffatome und/oder Schwefelatome und/oder ein oder mehrere substituierte oder unsubstituierte Iminogruppen unterbrochenes $C_2$-$C_{18}$-Alkyl, $C_6$-$C_{12}$-Aryl, $C_5$-$C_{12}$-Cycloalkyl, ein fünf- bis sechsgliedriger Sauerstoff-, Stickstoff- und/oder Schwefelatome aufweisender Heterocyclus,
wobei zwei der genannten Reste miteinander unter Ausbildung eines ungesättigten oder gesättigten Ringsegments verknüpft sein können, das gegebenenfalls durch ein oder mehrere Sauerstoff- und/oder Schwefelatome und/oder ein oder mehrere substituierte oder unsubstituierte Iminogruppen unterbrochen sein kann, wobei das Ringsegment durch funktionelle Gruppen, Aryl-, Alkyl-, Aryloxy-, Alkyloxy-, Halogen-, Heteroatom- und/oder Heterocyclus-Reste substituiert sein kann,
und wobei $R_4$ darüber hinaus ausgewählt sein kann aus der Gruppe der Reste bestehend aus $C_1$-$C_{18}$-Alkyloyl, $C_1$-$C_{18}$-Alkyloxycarbonyl, $C_5$-$C_{12}$-Cycloalkylcarbonyl und $C_6$-$C_{12}$-Aryloyl, wobei die Mitglieder der genannten Gruppe jeweils durch einen oder mehrere funktionelle Gruppen, Aryl-, Alkyl-, Aryloxy-, Alkyloxy-, Halogen-, Heteroatome und/oder Heterocyclus-Reste substituiert sein können, wobei sich die Angaben $C_1$-$C_{18}$, $C_5$-$C_{12}$, bzw. $C_6$-$C_{12}$ auf die Alkylkette beziehen.

Darin bedeutet der Begriff funktionelle Gruppen die Gruppe der nachstehenden funktionellen Gruppen: Aryl-, Alkyl-, Aryloxy-, Alkyloxy-, Halogen-, Heteroatom- und/oder Heterocyclen-substituiertes $C_1$-$C_{18}$-Alkyl, beispielsweise Methyl, Ethyl, Propyl, Isopropyl, n-Butyl, sec-Butyl, tert.-Butyl, Pentyl, Hexyl, Heptyl, Octyl, 2-Etylhexyl, 2,4,4-Trimethylpentyl, Decyl, Dodecyl, Tetradecyl, Heptadecyl, Octadecyl, 1,1-Dimethylpropyl, 1,1-Dimethylbutyl, 1,1,3,3-Tetramethylbutyl, Benzyl, 1-Phenylethyl, 2-Phenylethyl, alpha-alpha-Dimethylbenzyl, Benzhydryl, p-Tolylmethyl, 1-(p-Butyl-phenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonethyl, 2-Ethoxycarbonylethyl, 2-Butoxycarbonylpropyl, 1,2-Di-(methoxycarbonyl)-ethyl, 2-Methoxyethyl, 2-Ethoxyethyl, 2-Butoxyethyl, Diethoxymethyl, Diethoxyethyl, 1,3-Dioxolan-2-yl, 1,3-Dioxan-2-yl, 2-Methyl-1,3-dioxolan-2-yl, 4-Methyl-1,3-dioxolan-2-yl, 2-Isopropoxyethyl, 2-Butoxypropyl, 2-Octyloxyethyl, Chlormethyl, 2-Chlorethyl, Trichlormethyl, Trifluormethyl, 1,1-Dimethyl-2-chlorethyl, 2-Methoxyisopropyl, 2-Ethoxyethyl, Butylthiomethyl, 2-Dodecylthioethyl, 2-Phenylthioethyl, 2,2,2-Trifluorethyl, 2-Hydroxyethyl, 2-Hydroxypropyl, 3-Hydroxypropyl, 4-Hydroxybutyl, 6-Hydroxyhexyl, 2-Aminoethyl, 2-Aminopropyl, 3-Aminopropyl, 4-Aminobutyl, 6-Aminohexyl, 2-Methylaminoethyl, 2-Methylaminopropyl, 3-Methylaminopropyl, 4-Methylaminobutyl, 6-Methylaminohexyl, 2-Dimethylaminoethyl, 2-Dimethylaminopropyl, 3-Dimethylaminopropyl, 4-Dimethylaminobutyl, 6-Dimethylaminohexyl, 2-Hydroxy-2,2-dimethylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl oder 6-Ethoxyhexyl, durch ein oder mehrere nicht benachbarte Sauerstoff- und/oder Schwefelatome und/oder ein oder mehrere substituierte oder unsubstituierte Iminogruppen unterbrochenes $C_2$-$C_{18}$-Alkyl, beispielsweise 5-Hydroxy-3-oxa-pentyl, 8-Hydroxy-3,6-dioxa-octyl, 11-Hydroxy-3,6,9-trioxa-undecyl, 7-Hydroxy-4-oxa-heptyl, 11-Hydroxy-4,8-dioxa-undecyl, 15-Hydroxy-4,8,12-trioxa-pentadecyl, 9-Hydroxy-5-oxa-nonyl, 14-Hydroxy-5,10-oxa-tetradecyl, 5-Methoxy-3-oxa-pentyl, 8-Methoxy-3,6-dioxa-octyl, H-Methoxy-3,6,9-trioxa-undecyl, 7-Methoxy-4-oxa-heptyl, 11-Methoxy-4,8-dioxa-undecyl, 15-Methoxy-4,8,12-trioxa-pentadecyl, 9-Methoxy-5-oxa-nonyl, 14-Methoxy-5,10-oxa-tetradecyl, 5-Ethoxy-3-oxapentyl, 8-Ethoxy-3,6-dioxa-octyl, 11-Ethoxy-3,6,9-trioxa-undecyl, 7-Ethoxy-4-oxa-heptyl, 11-Ethoxy-4,8-dioxa-undecyl, 15-Ethoxy-4,8,12-trioxa-pentadecyl, 9-Ethoxy-5-oxa-nonyl und 14-Ethoxy-5,10-oxa-tetradecyl.

Bilden zwei Reste miteinander einen Ring aus, so können diese Reste gemeinsam vorzugsweise bedeuten 1,3-Propylen, 1,4-Butylen, 2-Oxa-1,3-propylen, 1-Oxa-1,3-propylen, 2-Oxa-1,3-propylen, 1-Oxa-1,3-propenylen, 1-Aza-1,3-propenylen, 1-$C_1$-$C_4$-Alkyl-1-aza-1,3-propenylen, 1,4-Buta-1,3-dienylen, 1-Aza-1,4-buta-1,3-dienylen oder 2-Aza-1,4-buta-1,3-dienylen.

Die Anzahl der Sauerstoff- und/oder Schwefelatome und/oder Iminogruppen in den bevorzugten Kationen der ionischen Flüssigkeit ist nicht beschränkt. Im Allgemeinen beträgt sie nicht mehr als 5 pro Rest, vorzugsweise nicht mehr als 4, insbesondere nicht mehr als 3. Weiterhin befindet sich zwischen zwei Heteroatomen mindestens ein Kohlenstoffatom, besonders bevorzugt mindestens zwei.

Bevorzugte Iminogruppen können beispielsweise sein Imino, Methylimino, iso-Propylimino, n-Butylimino oder tert-Butylimino.

Weiterhin bedeutet der Begriff funktionelle Gruppen die Gruppe der nachstehenden funktionellen Gruppen: Carboxy, Carboxamid, Hydroxy, Di-($C_1$-$C_4$-Alkyl)amino, $C_1$-$C_4$-Alkyloxycarbonyl, Cyano, $C_1$-$C_4$-Alkyloxy, durch funktionelle Gruppen-, Aryl-, Alkyl-, Aryloxy-, Alkyloxy-, Halogen-, Heteroatome- und/oder Heterocyclen-substituiertes $C_6$-$C_{12}$-Aryl, beispielsweise Phenyl, Tolyl, Xylyl, alpha-Naphthyl, beta-Naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, iso-Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlornaphthyl, Ethoxynaphthyl, 2,6-Dimethylphenyl, 2,4,6-Trimethylphenyl, 2,6-Diethoxyphenyl, 2,6-Dichlorphenyl, 4-Bromphenyl, 2- oder 4-Nitrophenyl, 2,4- oder 2,6-Dinitrophenyl, 4-Dimethylaminophenyl, 4-Acetylphenyl, Methoxyethylphenyl oder Ethoxyethylphenyl, durch funktionelle Gruppen-, Aryl-, Alkyl-, Aryloxy-, Alkyloxy-, Halogen-, Heteroatome- und/oder Heterocyclen-substituiertes $C_5$-$C_{12}$-Cycloalkyl, beispielsweise Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Methoxycyclohexyl, Dimethoxycyclohexyl, Diethoxycyclohexyl, Butylthiocyclohexyl, Chlorcyclohexyl, Dichlorcyclohexyl oder Dichlorcyclopentyl, gesättigte oder ungesättigte bicyclische Systeme, z.B. Norbornyl oder Norbornenyl, ein fünf- bis sechsgliedriger, Sauerstoff-, Stickstoff- und/oder Schwefelatome aufweisender Heterocyclus, beispielsweise Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, Isopropylthiophenyl oder tert.-Butylthiophenyl, und ein $C_1$-$C_4$-Alkyl, beispielsweise Methyl, Ethyl, Propyl, Isopropyl, n-Butyl, sec-Butyl oder tert.-Butyl.

$C_1$-$C_{18}$-Alkyloyl (Alkylcarbonyl) kann beispielsweise sein Acetyl, Propionyl, n-Butyloyl, sec-Butyloyl, tert.-Butyloyl, 2-Etylhexylcarbonyl, Decanoyl, Dodecanoyl, Chloracetyl, Trichloracetyl oder Trifluoracetyl.

$C_1$-$C_{18}$-Alkyloxycarbonyl kann beispielsweise sein Methyloxycarbonyl, Ethyloxycarbonyl, Propyloxycarbonyl, Isopropyloxycarbonyl, n-Butyloxycarbonyl, sec-Butyloxycarbonyl, tert.-Butyloxycarbonyl, Hexyloxycarbonyl, 2-Etylhexyloxycarbonyl oder Benzyloxycarbonyl.

$C_5$-$C_{12}$-Cycloalkylcarbonyl kann beispielsweise sein Cyclopentylcarbonyl, Cyclohexylcarbonyl oder Cyclododecylcarbonyl.

$C_5$-$C_{12}$-Aryloyl (Arylcarbonyl) kann beispielsweise sein Benzoyl, Toluyl, Xyloyl, alpha-Naphthoyl, beta-Naphthoyl, Chlorbenzoyl, Dichlorbenzoyl, Trichlorbenzoyl oder Trimethylbenzoyl.

Bevorzugt sind $R_1$, $R_2$, $R_3$, $R_5$, $R_6$, $R_7$, $R_8$, $R_9$ und $R_{10}$ jeweils unabhängig voneinander, Wasserstoff, Methyl, Ethyl, n-Butyl, 2-Hydroxyethyl, 2-Cyanoethyl, 2-(Methoxycarbonyl)-ethyl, 2-Ethoxycarbonyl-ethyl, 2-n-Butoxycarbonyl-ethyl, Dimethylamino, Diethylamino oder Chlor.

Bevorzugt ist $R_4$ Methyl, Ethyl, n-Butyl, 2-Hydroxyethyl, 2-Cyanoethyl, 2-Methoxycarbonyl-ethyl, 2-Ethoxycarbonyl-ethyl, 2-n-Butoxycarbonyl-ethyl, Acetyl, Propionyl, t-Butyryl, Methoxycarbonyl, Ethoxycarbonyl oder n-Butoxycarbonyl.

Besonders bevorzugte Ammoniumionen (IL-1) sind solche, bei denen unabhängig voneinander $R_4$ unter Acetyl, Methyl, Ethyl oder n-Butyl und $R_1$, $R_2$ und $R_3$ unter Methyl, Ethyl, n-Butyl, 2-Hydroxyethyl, Benzyl oder Phenyl ausgewählt sind.

Besonders bevorzugte Phosphoniumionen (IL-2) sind solche, bei denen unabhängig voneinander $R_4$ unter Acetyl, Methyl, Ethyl oder n-Butyl und $R_1$, $R_2$ und $R_3$ unter Phenyl, Phenoxy, Ethoxy und n-Butoxy ausgewählt sind.

Besonders bevorzugte Pyrrolidiniumionen (IL-3) sind solche, bei denen unabhängig voneinander $R_3$ und $R_4$ unter Acetyl, Methyl, Ethyl oder n-Butyl ausgewählt sind und alle anderen Reste Wasserstoff bedeuten.

Besonders bevorzugte 1-Pyrazoliniumionen (IL-4) sind solche, bei denen unabhängig voneinander alle Reste bis auf $R_4$ unter Wasserstoff oder Methyl und $R_4$ unter Acetyl, Methyl, Ethyl oder n-Butyl ausgewählt sind.

Besonders bevorzugte 2-Pyrazoliniumionen (IL-5) sind solche, bei denen unabhängig voneinander $R_5$ unter Wasserstoff, Methyl, Ethyl oder Phenyl, $R_4$ unter Acetyl, Methyl, Ethyl oder n-Butyl und die übrigen Reste unter Wasserstoff oder Methyl ausgewählt sind.

Besonders bevorzugte 3-Pyrazoliniumionen (IL-6) sind solche, bei denen unabhängig voneinander $R_3$ und $R_5$ unter Wasserstoff, Methyl, Ethyl oder Phenyl, $R_4$ unter Acetyl, Methyl, Ethyl oder n-Butyl und die verbleibenden Reste unter Wasserstoff oder Methyl ausgewählt sind.

Besonders bevorzugte 1H-Pyrazoliumionen (IL-7) sind solche, bei denen unabhängig voneinander $R_5$ unter Wasserstoff, Methyl oder Ethyl, $R_1$, $R_2$ und $R_3$ unter Wasserstoff oder Methyl und $R_4$ unter Acetyl, Methyl, Ethyl oder n-Butyl ausgewählt sind.

Besonders bevorzugte 3H-Pyrazoliumionen (IL-8) sind solche, bei denen unabhängig voneinander $R_2$ unter Wasserstoff, Methyl oder Ethyl, $R_1$, $R_3$ und $R_5$ unter Wasserstoff oder Methyl und $R_4$ unter Acetyl, Methyl, Ethyl oder n-Butyl ausgewählt sind.

Besonders bevorzugte 4H-Pyrazoliumionen (IL-9) sind solche, bei denen unabhängig voneinander $R_1$ $R_2$, $R_3$ und $R_5$ unter Wasserstoff oder Methyl und $R_4$ unter Acetyl, Methyl, Ethyl oder n-Butyl ausgewählt sind.

Besonders bevorzugte Imidazoliniumionen (IL-10) sind solche, bei denen unabhängig voneinander $R_5$ oder $R_6$ unter Wasserstoff, Methyl oder Ethyl, $R_4$ unter Acetyl, Methyl, Ethyl oder n-Butyl und die übrigen Reste unter Wasserstoff oder Methyl ausgewählt sind.

Besonders bevorzugte Imidazoliniumionen (IL-11) sind solche, bei denen unabhängig voneinander $R_5$, $R_6$ oder $R_7$ unter Wasserstoff, Methyl oder Ethyl, $R_4$ unter Acetyl, Methyl, Ethyl oder n-Butyl und die übrigen Reste unter Wasserstoff oder Methyl ausgewählt sind.

Besonders bevorzugte Imidazoliniumionen (IL-12) sind solche, bei denen unabhängig voneinander $R_3$ oder $R_7$ unter Wasserstoff, Methyl, Ethyl, n-Butyl oder Phenyl, $R_4$ unter Acetyl, Methyl, Ethyl oder n-Butyl und $R_5$ oder $R_6$ unter Wasserstoff, Methyl oder Ethyl und $R_1$ oder $R_2$ unter Wasserstoff oder Methyl ausgewählt sind.

Besonders bevorzugte Imidazoliumionen (IL-13) sind solche, bei denen unabhängig voneinander $R_1$ ausgewählt ist aus der Gruppe bestehend aus Methyl, Ethyl, n-Propyl, n-Butyl, n-Pentyl, n-Octyl, n-Decyl, n-Dodecyl, 2-Hydroxyethyl und 2-Cyanoethyl, $R_4$ aus Acetyl, Methyl, Ethyl oder n-Butyl und die übrigen Reste unabhängig voneinander aus Wasserstoff, Methyl oder Ethyl.

Besonders bevorzugte 1,2,4-Triazoliumionen (IL-14) und (IL-15) sind solche, bei denen unabhängig voneinander $R_1$ oder $R_2$ bzw. $R_1$ oder $R_3$ unter Wasserstoff, Methyl, Ethyl oder Phenyl, $R_4$ unter Acetyl, Methyl, Ethyl oder n-Butyl und $R_3$ bzw. $R_2$ unter Wasserstoff, Methyl oder Phenyl ausgewählt sind. Besonders bevorzugte 1,2,3-Triazoliumionen (IL-16) und (IL-17) sind solche, bei denen unabhängig voneinander $R_3$ bzw. $R_1$ unter Wasserstoff, Methyl oder Ethyl, $R_4$ unter Acetyl, Methyl, Ethyl oder n-Butyl und $R_1$ oder $R_2$ bzw. $R_2$ oder $R_3$ unter Wasserstoff oder Methyl ausgewählt sind oder $R_1$ und $R_2$ bzw. $R_2$ und $R_3$ 1,4-Buta-1,3-dienylen und alle anderen Reste Wasserstoff sind.

Besonders bevorzugte Thiazoliumionen (IL-18) oder Oxazoliumionen (IL-19) sind solche, bei denen unabhängig voneinander $R_1$ unter Wasserstoff, Methyl, Ethyl oder Phenyl, $R_4$ unter Acetyl, Methyl, Ethyl oder n-Butyl und $R_2$ oder $R_3$ unter Wasserstoff oder Methyl ausgewählt sind.

Besonders bevorzugte Pyridiniumionen (IL-20) sind solche, bei denen einer der Reste $R_1$, $R_2$, $R_3$, $R_5$ und $R_6$ Methyl, Ethyl oder Chlor ist, $R_4$ Acetyl, Methyl, Ethyl oder n-Butyl ist und alle anderen Reste Wasserstoff sind, oder $R_1$ Dimethylamino, $R_4$ Acetyl, Methyl, Ethyl oder n-Butyl und alle anderen Reste Wasserstoff sind oder $R_4$ Acetyl, Methyl, Ethyl oder n-Butyl und alle anderen Reste Wasserstoff sind oder $R_2$ Carboxy oder Carboxamid, $R_4$ Acetyl, Methyl, Ethyl oder n-Butyl und alle anderen Reste Wasserstoff sind oder $R_2$ und $R_3$ oder $R_2$ und $R_1$ 1,4-Buta-1,3-dienylen, $R_4$ Acetyl, Methyl, Ethyl oder n-Butyl und alle anderen Reste Wasserstoff sind.

Besonders bevorzugte Pyrimidiniumionen (IL-21) sind solche, bei denen $R_1$, $R_4$ und $R_5$ Wasserstoff oder Methyl, $R_4$ Acetyl, Methyl, Ethyl oder n-Butyl und $R_3$ Wasserstoff, Methyl oder Ethyl sind, oder $R_2$ und $R_5$ Methyl, $R_1$ Wasserstoff und $R_3$ Wasserstoff, Methyl oder Ethyl und $R_4$ Acetyl, Methyl, Ethyl oder n-Butyl sind.

Besonders bevorzugte Pyridaziniumionen (IL-22) sind solche, bei denen einer der Reste $R_1$, $R_2$, $R_3$ und $R_5$ Methyl oder Ethyl, $R_4$ Acetyl, Methyl, Ethyl oder n-Butyl und alle anderen Reste Wasserstoff oder $R_4$ Acetyl, Methyl, Ethyl oder n-Butyl, und alle anderen Reste Wasserstoff sind.

Besonders bevorzugte Pyraziniumionen (IL-23) sind solche, bei denen $R_1$, $R_2$, $R_3$ und $R_5$ alle Methyl und $R_4$ Acetyl, Methyl, Ethyl oder n-Butyl oder $R_4$ Acetyl, Methyl, Ethyl oder n-Butyl und alle anderen Reste Wasserstoff sind.

Von den zuvor erwähnten Kationengruppen IL-1 bis IL-23 sind die genannten Ammonium-, Phosphonium-, Pyridinium- und Imidazoliumionen besonders bevorzugt.

Ganz besonders bevorzugt sind als Kationen 1,2-Dimethylpyridinium, 1-Methyl-2-ethylpyridinium, 1-Methyl-2-ethyl-6-methylpyridinium, N-Methylpyridinium, 1-Butyl-2-methylpyridinium, 1-Butyl-2-ethylpyridinium, 1-Butyl-2-ethyl-6-methylpyridinium, N-Butylpyridinium, 1-Butyl-4-methylpyridinium, 1,3-Dimethylimidazolium, 1,2,3-Trimethylimidazolium, 1-n-Butyl-3-methylimidazolium, 1,3,4,5-Tetramethylimidazolium, 1,3,4-Trimethylimidazolium, 2,3-Dimethylimidazolium, 1-Butyl-2,3-dimethylimidazolium, 3,4-Dimethylimidazolium, 2-Ethyl-3,4-dimethylimidazolium, 3-Methyl-2-ethylimidazol, 3-Butyl-1-methylimidazolium, 3-Butyl-1-ethylimidazolium, 3-Butyl-1,2-dimeraylimidazolium, 1,3-Din-Butylimidazolium, 3-Butyl-1,4,5-Trimethylimidazolium, 3-Butyl-1,4-Dimethylimidazolium, 3-Butyl-2-methylimidazolium, 1,3-Dibutyl-2-methylimidazolium, 3-Butyl-4-methylimidazolium, 3-Butyl-2-ethyl-4-methylimidazolium und 3-Butyl-2-ethylimidazolium, 1-Methyl-3-Octylimidazolium, 1-Decyl-3-methylimidazolium.

Insbesondere bevorzugt sind 1-Butyl-4-methylpyridinium, 1-n-Butyl-3-methylimidazolium und 1-n-Butyl-3-ethylimidazolium.

Auch möglich sind Kationen, die von Diazabicyclononen oder Diazabicycloundecen abgeleitet werden.

Analog den obigen Ausführungen kann das Anion der ionischen Flüssigkeit beliebiger Natur sein. Es ist jedoch bevorzugt, wenn das Anion der ionischen Flüssigkeit ausgewählt ist aus der Gruppe bestehend aus $F^-$, $Cl^-$, $Br^-$, $I^-$, $PF_6^-$, $BF_4^-$, Alkylsulfat, vorzugsweise ein $C_1$ bis $C_{18}$ Alkylsulfat, Ethersulfat, Acetat, Trifluoracetat, Triflat, Sulfat, Hydrogensulfit, Methylsulfat, Ethylsulfat, Sulfit, Hydrogensulfit, Aluminiumchloride, vorzugsweise $AlCl_4^-$, $Al_2Cl_7^-$ oder $Al_3Cl_{10}^-$, Aluminiumtribromid, Nitrit, Nitrat, Metallkomplexe, beispielsweise Metallhalogenide wie Kupferchlorid $CuCl_2^-$, Phosphate, Phosphat, Hydrogenphosphat, Dihydrogenphosphat, Carbonat, Hydrogencarbonat, Sulfonat, Tosylat, Bis(trifluormethylsulfonyl)imid, Cyanid und Isocyanat.

Unter Ethersulfate werden vorliegende Verbindungen der allgemeinen Formel

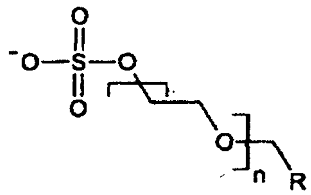

verstanden, worin n eine ganze Zahl von 1 bis 8 ist und R ein Alkylrest von $C_1$ bis $C_{18}$ ist.

Es wurde festgestellt, dass die vorteilhaften Effekte des erfindungsgemäßen Katalysators dann besonders deutlich eintreten, wenn die mittlere Dicke der IL-Beschichtung zumindest gleich der Dicke einer einzelnen Ionenlage der ionischen Flüssigkeit ist. Gemäß einer bevorzugten Ausführungsform des erfindungsgemäßen Katalysators weist die IL-Beschichtung eine mittlere Dicke auf, die gleich der Dicke einer einzelnen Ionenlage der ionischen Flüssigkeit ist oder die größer ist als die Dicke einer einzelnen Ionenlage der ionischen Flüssigkeit, vorzugsweise größer als die Dicke von 1,1 Ionenlagen, bevorzugt größer als die Dicke von 1,2 Ionenlagen, weiter bevorzugt größer als die Dicke von 1,3 Ionenlagen, besonders bevorzugt größer als die Dicke von 1,4 Ionenlagen und weiter besonders bevorzugt größer als die Dicke von 1,5 Ionenlagen. Dabei wird unter einer Ionenlage im Rahmen der vorliegenden Erfindung eine einlagige Schicht eines Anion/Kation-Ionenpaares der ionischen Flüssigkeit verstanden.

Weiter bevorzugt ist, dass die IL-Beschichtung eine mittlere Dicke aufweist, die größer ist als die Dicke von 1,6 Ionenlagen der ionischen Flüssigkeit, vorzugsweise-größer als die Dicke von 1,7 Ionenlagen, bevorzugt größer als die Dicke von 1,8 Ionenlagen, weiter bevorzugt größer als die Dicke von 1,9 Ionenlagen, besonders bevorzugt größer als die Dicke von zumindest 2,0 Ionenlagen und weiter besonders bevorzugt größer als die Dicke von zumindest 2,1 Ionenlagen.

Ferner kann es bevorzugt sein, dass die IL-Beschichtung eine mittlere Dicke aufweist, die größer ist als die Dicke von 2,2 Ionenlagen der ionischen Flüssigkeit, vorzugsweise größer als die Dicke von 2,3 Ionenlagen, bevorzugt größer als die Dicke von 2,4 Ionenlagen, weiter bevorzugt größer als die Dicke von 2,5 Ionenlagen, besonders bevorzugt größer als die Dicke von zumindest 2,6 Ionenlagen und weiter besonders bevorzugt größer als die Dicke von zumindest 2,7 Ionenlagen.

Darüber hinaus kann es bevorzugt sein, dass die IL-Beschichtung eine mittlere Dicke aufweist, die größer ist als die Dicke von 2,8 Ionenlagen der ionischen Flüssigkeit, vorzugsweise größer als die Dicke von 2,9 Ionenlagen, bevorzugt größer als die Dicke von 3,0 Ionenlagen, weiter bevorzugt größer als die Dicke von 3,1 Ionenlagen, besonders bevorzugt größer als die Dicke von zumindest 3,2 Ionenlagen und weiter besonders bevorzugt größer als die Dicke von zumindest 3,3 Ionenlagen.

Auch kann es bevorzugt sein, dass die IL-Beschichtung eine mittlere Dicke aufweist, die größer ist als die Dicke von 3,4 Ionenlagen der ionischen Flüssigkeit, vorzugsweise größer als die Dicke von 3,5 Ionenlagen, bevorzugt größer als die Dicke von 3,6 Ionenlagen, weiter bevorzugt größer als die Dicke von 3,7 Ionenlagen, besonders bevorzugt größer als die Dicke von zumindest 3,8 Ionenlagen und weiter besonders bevorzugt größer als die Dicke von zumindest 3,9 Ionenlagen.

Grundsätzlich kann die IL-Beschichtung eine sehr große Dicke aufweisen. Wird ab einer bestimmten Schichtdicke aber die Schichtdicke noch weiter erhöht, so führt dies lediglich zu einer Abnahme der Aktivität des Katalysators bei gleich bleibender Selektivität. In einer anderen Ausführungsform des erfindungsgemäßen Katalysators ist es bevorzugt, dass die IL-Beschichtung eine mittlere Dicke aufweist, die kleiner ist als die Dicke von 40 Ionenlagen der ionischen Flüssigkeit, vorzugsweise kleiner als die Dicke von 38 Ionenlagen, bevorzugt kleiner als die Dicke von 36 Ionenlagen, weiter bevorzugt kleiner als die Dicke von 34 Ionenlagen, besonders bevorzugt kleiner als die Dicke von 32 Ionenlagen und weiter besonders bevorzugt kleiner als die Dicke von 30 Ionenlagen.

Es kann auch bevorzugt sein, dass die IL-Beschichtung eine mittlere Dicke aufweist, die kleiner ist als die Dicke von 28 Ionenlagen der ionischen Flüssigkeit, vorzugsweise kleiner als die Dicke von 26 Ionenlagen, bevorzugt kleiner als die Dicke von 24 Ionenlagen, weiter bevorzugt kleiner als die Dicke von 22 Ionenlagen, besonders bevorzugt kleiner als die Dicke von 20 Ionenlagen und weiter besonders bevorzugt kleiner als die Dicke von 19 Ionenlagen.

Auch kann bevorzugt vorgesehen sein, dass die IL-Beschichtung eine mittlere Dicke aufweist, die kleiner ist als die Dicke von 18 Ionenlagen der ionischen Flüssigkeit, vorzugsweise kleiner als die Dicke von 17 Ionenlagen, bevorzugt kleiner als die Dicke von 16 Ionenlagen, weiter bevorzugt kleiner als die Dicke von 15 Ionenlagen, besonders bevorzugt kleiner als die Dicke von 14 Ionenlagen und weiter besonders bevorzugt kleiner als die Dicke von 13 Ionenlagen.

Ferner kann bevorzugt sein, dass die IL-Beschichtung eine mittlere Dicke aufweist, die kleiner ist als die Dicke von 12 Ionenlagen der ionischen Flüssigkeit, vorzugsweise kleiner als die Dicke von 11 Ionenlagen, bevorzugt kleiner als die Dicke von 10 Ionenlagen, weiter bevorzugt kleiner als die Dicke von 9,5 Ionenlagen, besonders bevorzugt kleiner als die Dicke von 9 Ionenlagen und weiter besonders bevorzugt kleiner als die Dicke von 8,8 Ionenlagen.

Darüber hinaus kann es bevorzugt sein, dass die IL-Beschichtung eine mittlere Dicke aufweist, die kleiner ist als die Dicke von 8,6 Ionenlagen der ionischen Flüssigkeit, vorzugsweise kleiner als die Dicke von 8,4 Ionenlagen, bevorzugt kleiner als die Dicke von 8,2 Ionenlagen, weiter bevorzugt kleiner als die Dicke von 8 Ionenlagen, besonders bevorzugt kleiner als die Dicke von 7,8 Ionenlagen und weiter besonders bevorzugt kleiner als die Dicke von 7,6 Ionenlagen.

Auch kann es bevorzugt vorgesehen sein, dass die IL-Beschichtung eine mittlere Dicke aufweist, die kleiner ist als die Dicke von 7,4 Ionenlagen der ionischen Flüssigkeit, vorzugsweise kleiner als die Dicke von 7,2 Ionenlagen, bevorzugt kleiner als die Dicke von 7,0 Ionenlagen, weiter bevorzugt kleiner als die Dicke von 6,8 Ionenlagen, besonders bevorzugt kleiner als die Dicke von 6,6 Ionenlagen und weiter besonders bevorzugt kleiner als die Dicke von 6,4 Ionenlagen.

Ferner kann bevorzugt sein, dass die IL-Beschichtung eine mittlere Dicke aufweist, die kleiner ist als die Dicke von 6,2 Ionenlagen der ionischen Flüssigkeit, vorzugsweise kleiner als die Dicke von 6 Ionenlagen, bevorzugt kleiner als die Dicke von 5,8 Ionenlagen, weiter bevorzugt kleiner als die Dicke von 5,6 Ionenlagen, besonders bevorzugt kleiner als die Dicke von 5,4 Ionenlagen und weiter besonders bevorzugt kleiner als die Dicke von 5,2 Ionenlagen.

Darüber hinaus kann es bevorzugt sein, dass die IL-Beschichtung eine mittlere Dicke aufweist, die kleiner ist als die Dicke von 5 Ionenlagen der ionischen Flüssigkeit, vorzugsweise kleiner als die Dicke von 4,8 Ionenlagen, bevorzugt kleiner als die Dicke von 4,6 Ionenlagen, weiter bevorzugt kleiner als die Dicke von 4,4 Ionenlagen, besonders bevorzugt kleiner als die Dicke von 4,2 Ionenlagen und weiter besonders bevorzugt kleiner als die Dicke von 4 Ionenlagen.

Entsprechend einer weiteren bevorzugten Ausführungsform des erfindungsgemäßen Verfahrens weist die IL-Beschichtung eine mittlere Dicke auf, die größer ist als die Dicke von einer Ionenlage der ionischen Flüssigkeit und die kleiner ist als die Dicke von 10 Ionenlagen der ionischen Flüssigkeit, vorzugsweise größer als die Dicke von 1,1 Ionenlagen und kleiner als die Dicke von 9,8 Ionenlagen, bevorzugt größer als die Dicke von 1,2 Ionenlagen und kleiner als die Dicke von 9,6 Ionenlagen, weiter bevorzugt größer als die Dicke von 1,3 Ionenlagen und kleiner als die Dicke von 9,4 Ionenlagen, mehr bevorzugt größer als die Dicke von 1,4 Ionenlagen und kleiner als die Dicke von 9,2 Ionenlagen, weiter mehr bevorzugt größer als die Dicke von 1,5 Ionenlagen und kleiner als die Dicke von 9 Ionenlagen und besonders bevorzugt größer als die Dicke von 1,6 Ionenlagen und kleiner als die Dicke von 8,8 Ionenlagen.

Gemäß einer anderen bevorzugten Ausführungsform des erfindungsgemäßen Katalysators ist vorgesehen, dass die IL-Beschichtung eine mittlere Dicke aufweist, die größer ist als die Dicke von 1,7 Ionenlagen der ionischen Flüssigkeit und die kleiner ist als die Dicke von 8,6 Ionenlagen der ionischen Flüssigkeit, vorzugsweise größer als die Dicke von 1,8 Ionenlagen und kleiner als die Dicke von 8,4 Ionenlagen, bevorzugt größer als die Dicke von 1,9 Ionenlagen und kleiner als die Dicke von 8,2 Ionenlagen, weiter bevorzugt größer als die Dicke von 2 Ionenlagen und kleiner als die Dicke von 8 Ionenlagen, mehr bevorzugt größer als die Dicke von 2,1 Ionenlagen und kleiner als die Dicke von 7,8 Ionenlagen, weiter mehr bevorzugt größer als die Dicke von 2,2 Ionenlagen und kleiner als die Dicke von 7,6 Ionenlagen und besonders bevorzugt größer als die Dicke von 2,3 Ionenlagen und kleiner als die Dicke von 7,4 Ionenlagen.

Bei einer anderen bevorzugten Ausführungsform des erfindungsgemäßen Katalysators ist vorgesehen, dass die IL-Beschichtung eine mittlere Dicke aufweist, die größer ist als die Dicke von 2,4 Ionenlagen der ionischen Flüssigkeit und die kleiner ist als die Dicke von 7,2 Ionenlagen der ionischen Flüssigkeit, vorzugsweise größer als die Dicke von 2,5 Ionenlagen und kleiner als die Dicke von 7 Ionenlagen, bevorzugt größer als die Dicke von 2,6 Ionenlagen und kleiner als die Dicke von 6,8 Ionenlagen, weiter bevorzugt größer als die Dicke von 2,7 Ionenlagen und kleiner als die Dicke von 6,6 Ionenlagen, mehr bevorzugt größer als die Dicke von 2,8 Ionenlagen und kleiner als die Dicke von 6,4 Ionenlagen, weiter mehr bevorzugt größer als die Dicke von 2,9 Ionenlagen und kleiner als die Dicke von 6,2 Ionenlagen und besonders bevorzugt größer als die Dicke von 3 Ionenlagen und kleiner als die Dicke von 6 Ionenlagen.

Es kann auch bevorzugt vorgesehen sein, dass die IL-Beschichtung eine mittlere Dicke aufweist, die größer ist als die Dicke von 3,1 Ionenlagen der ionischen Flüssigkeit und die kleiner ist als die Dicke von 5,8 Ionenlagen der ionischen Flüssigkeit, vorzugsweise größer als die Dicke von 3,2 Ionenlagen und kleiner als die Dicke von 5,6 Ionenlagen, bevorzugt größer als die Dicke von 3,3 Ionenlagen und kleiner als die Dicke von 5,4 Ionenlagen, weiter bevorzugt größer

als die Dicke von 3,4 Ionenlagen und kleiner als die Dicke von 5,2 Ionenlagen, mehr bevorzugt größer als die Dicke von 3,5 Ionenlagen und kleiner als die Dicke von 5 Ionenlagen, weiter mehr bevorzugt größer als die Dicke von 3,6 Ionenlagen und kleiner als die Dicke von 4,8 Ionenlagen und besonders bevorzugt größer als die Dicke von 3,7 Ionenlagen und kleiner als die Dicke von 4,6 Ionenlagen.

Besonders bevorzugt ist es, dass die IL-Beschichtung eine mittlere Dicke aufweist, die größer/gleich ist als/wie die Dicke von 1 Ionenlage/n der ionischen

Die vorgenannten oxidischen Trägermaterialien können beispielsweise in Form von Mischoxiden oder definierter Zusammensetzung bevorzugt eingesetzt werden, beispielsweise $TiO_2$, $SiO_2$, $Al_2O_3$, $ZrO_2$, MgO, $SiC_2$ oder ZnO. Weiterhin können vorzugsweise Ruße, Acetylenschwarz, Kohle, Graphit, Hydrotalcite oder weitere, dem Fachmann an sich bekannte Trägermaterialien in verschiedenen möglichen Modifikationen eingesetzt sein. Die Trägermaterialien können vorzugsweise etwa mit Alkali- oder Erdalkalimetallen oder auch mit Phosphor-, Halogenid- und/oder Sulfatsalzen dotiert sein. Im Allgemeinen werden die Säure-/Baseeigenschaften durch solche Dotierungen modifiziert, was sich positiv auf die katalytischen Eigenschaften auswirken kann. Die zuvor genannten hydrieraktiven Metalle können durch jedes geeignete Verfahren auf dem Träger aufgebracht sein, beispielsweise durch Imprägnieren, Ionenaustausch, Copräzipitation, z.B. gemeinsame Fällung mit dem Träger, Fällung auf einen vorgelegten Träger, lonenaustausch oder Chemical Vapour Deposition (CVD).

Die Erfindung betrifft ferner ein Verfahren zur Herstellung eines porösen heterogenen übergangsmetallkatalysators gemäß Anspruch 9, dessen innere Oberfläche mit einer ionischen Flüssigkeit beschichtet ist, umfassend die folgenden Schritte:

1. a) das Lösen einer ionischen Flüssigkeit in einem geeigneten Lösemittel;
2. b) das Inkontaktbringen eines porösen heterogenen übergangsmetallkatalysators mit dem die ionische Flüssigkeit enthaltenden Lösemittel gemäß Schritt a);
3. c) das Entfernen des Lösemittels.

Die Erfindung betrifft ferner einen Katalysator erhältlich durch das vorstehende Verfahren mit den Schritten a) bis c).

Die Erfindung betrifft darüber hinaus ein Verfahren zur Herstellung eines porösen heterogenen übergangsmetallkatalysators, dessen innere Oberfläche mit einer ionischen Flüssigkeit beschichtet ist, umfassend die nachfolgenden Schritte:

1. a) das Lösen einer ionischen Flüssigkeit in einem geeigneten Lösemittel;
2. b) das Einbringen des Übergangsmetallkatalysators in eine Kammer, in der eine durch eine Besaugung der Kammer bewirkte Strömung sowie ein Unterdruck herrscht;
3. c) das Einleiten des die ionische Flüssigkeit enthaltenden Lösemittels in die Kammer;
4. d) das Bilden eines Gemisches aus Gas und dem die ionische Flüssigkeit enthaltenden Lösemittel;
5. e) das kontinuierliche Durchführen des Gemisches aus Schritt d) durch den Katalysator;
6. f) das Entfernen des Lösemittels.

Das eingesetzte Gas ist dabei ein Schutzgas wie beispielsweise ein Edelgas oder aber auch Stickstoff. Das Gas wird eingesetzt, um eine Zersetzung der ionischen Flüssigkeit durch Luftbestandteile zu verhindern.

Bevorzugt ist es, dass der durch die Besaugung bewirkte Unterdruck in der Kammer kleiner ist als 800 mbar.

Weiter ist es bevorzugt, dass das Verfahren bei einem Druck von 300 mbar bis 500 mbar durchgeführt wird.

Auch kann es bevorzugt sein, dass beim Bilden des Gemisches aus Gas und dem die ionische Flüssigkeit enthaltenden Lösemittel Tropfen mit einem mittleren Durchmesser von 1 Mikrometer bis 900 Mikrometer gebildet werden.

Die Erfindung betrifft ferner einen Katalysator erhältlich durch das vorstehende Verfahren mit den Schritten a) bis f).

Die Erfindung betrifft ferner die Verwendung des erfindungsgemäßen Katalysators zur selektiven Hydrierung von ungesättigten Gruppen mehrfach ungesättigten Verbindungen, insbesondere zur Hydrierung von aromatischen Verbindungen zu Cycloolefinen, zur Hydrierung von Acetylen zu Ethylen, zur Hydrierung von Diolefinen in Monolefine, insbesondere zur Hydrierung von Butadien zu 1-Buten und/oder 2-Buten, zur Hydrierung von Phenylacetylen zu Styrol, zur Hydrierung von Methylacetylen und Propadien zu Propen, zur Hydrierung von Olefinen und Diolefinen in aromatischen Strömen ohne Verlust an Aromaten oder zur Hydrierung von Cyclododecatrien zu Cyclododecen.

Die nachstehenden Beispiele dienen im Zusammenhang mit der Beschreibung der Zeichnung der Erläuterung der Erfindung.

Es zeigen:

| | |
|---|---|
| Fig. 1: | Eine graphische Darstellung der mittels BET- und Hg-Porosimetrie-Messungen ermittelten Abhängigkeit des Porenvolumens vom Porendurchmesser eines geträgerten Nickelkatalysators ohne IL-Beschichtung (Kurve 1a) als Vergleichsbeispiel, eines mit einer ionischen Flüssigkeit beschichteten Nickelkatalysators gemäß der Erfindung (Kurve 2a) und des erfindungsgemäßen Katalysators (Kurve 3a), nachdem dieser in einer Hydrierungsreaktion eingesetzt wurde; |
| | |
| Fig. 2: | eine graphische Darstellung der mittels BET- und Hg-Porosimetrie-Messungen ermittelten Abhängigkeit der inneren Oberfläche vom Porendurchmesser des geträgerten Nickelkatalysators ohne IL-Beschichtung (Kurve 1b), des mit einer ionischen Flüssigkeit beschichteten Nickelkatalysators (Kurve 2b) und des erfindungsgemäßen Katalysators (Kurve 3b), nachdem dieser in einer Hydrierungsreaktion eingesetzt wurde; |
| | |
| Fig. 3: | Abhängigkeit der Umsatzrate von der modifizierten Versuchszeit des Katalysators ohne IL-Beschichtung (Kurve 1c) und des erfindungsgemäßen Katalysators (Kurve 2c) bei der Umsetzung von Cyclooctadien mit Wasserstoff; |
| | |
| Fig. 4: | Restanteil an Cyclooctadien (Kurve 1d) und Ausbeute an cis-Cycloocten (Kurve 1e) und Cyclooctan (Kurve 1f) in Abhängigkeit von der modifizierten Versuchszeit bei der Hydrierung von Cyclooctadien mittels des geträgerten Nickelkatalysators ohne IL-Beschichtung; |
| | |
| Fig. 5: | Selektivität bezüglich des cis-Cyclooctens (Kurve 1g) und des Cyclooctans (Kurve 1h) in Abhängigkeit vom Cyclooctadien-Umsatz bei der Hydrierung von Cyclooctadien mittels des geträgerten Nickelkatalysators ohne IL-Beschichtung; |
| | |
| Fig. 6: | Restanteil an Cyclooctadien (Kurve 2d) und Ausbeute an cis-Cycloocten (Kurve 2e) und Cyclooctan (Kurve 2f) in Abhängigkeit von der modifizierten Versuchszeit bei der Hydrierung von Cyclooctadien mittels des mit einer ionischen Flüssigkeit beschichteten geträgerten Nickelkatalysators; |
| | |
| Fig. 7: | Selektivität bezüglich des cis-Cyclooctens (Kurve 2g) und des Cyclooctans (Kurve 2h) in Abhängigkeit vom Cyclooctadien-Umsatz bei der Hydrierung von Cyclooctadien mittels des mit einer ionischen Flüssigkeit beschichteten geträgerten Nickelkatalysators. |

**Beispiel 1:**

Ein geträgerter Nickelkatalysator der Firma Süd-Chemie AG mit der Bezeichnung G-33 RS, der im Handel in Form von Tabletten von 6 X 6 mm angeboten wird, wurde mittels einer Kugelmühle zu einem Pulver vermahlen. Von dem Mahlgut wurden die Partikel mit einem Durchmesser im Bereich von 125-250 Mikrometer abgetrennt und diese einer auf eine Temperatur von 300°C erwärmten, reduzierend wirkenden Wasserstoffatmosphäre zur Aktivierung des Nickelmetalls ausgesetzt.

Der derartig aktivierte unbeschichtete Nickelkatalysator (Katalysator ohne IL-Beschichtung) wurde in den nachfolgenden Messungen und Reaktionen als Vergleichsbeispiel eingesetzt.

**Beispiel 2:**

Zur Herstellung des erfindungsgemäßen Katalysators wurden 0,8145 g des wie vorstehend unter Beispiel 1 hergestellten unbeschichteten aktivierten Nickelkatalysators in einer Lösung aus 100 ml $CH_2Cl_2$ und 0,05 g 1-Butyl-3-methyl-imidazolium-Octylsulfat als ionische Flüssigkeit unter einer Stickstoff-Schutzgasatmosphäre suspendiert und das Lösemittel $CH_2Cl_2$ unter Vakuum am Rotationsverdampfer abgedampft.

**Beispiel 3:**

Das Porensystem des aktivierten Nickelkatalysators ohne IL-Beschichtung gemäß Beispiel 1 und des erfindungsgemäßen, mit einer IL-Beschichtung versehenen Nickelkatalysators gemäß Beispiel 2 wurden mittels BET- und Hg-Porosimetrie-Messungen charakterisiert.

Die Fig. 1 zeigt die Abhängigkeit des Porenvolumens des beschichteten und des unbeschichteten Katalysators vom Porendurchmesser. Die Kurvenverläufe 1a und 2a des unbeschichteten bzw. des beschichteten Katalysators lassen erkennen, dass die Poren des beschichteten Katalysators weitgehend unabhängig von ihrem Durchmesser mit der ionischen Flüssigkeit mit im Wesentlichen gleichmäßiger Dicke beschichtet sind. Aus den Daten konnte ermittelt werden, dass die IL-Beschichtung ca. 22 Vol-% des ursprünglichen Volumens des unbeschichteten Nickelkatalysators einnimmt.

Die Fig. 2 zeigt die Abhängigkeit der inneren Oberfläche des unbeschichteten und des beschichteten Nickelkatalysators vom Porendurchmesser. Den Kurvenverläufen 1b und 2b des unbeschichteten bzw. des beschichteten Nickelkatalysators lässt sich entnehmen, dass durch die IL-Beschichtung im Vergleich zum unbeschichteten Katalysator die innere Oberfläche des beschichteten Katalysators um etwa 45 % vermindert ist. Hieraus lässt sich eine mittlere Dicke der IL-Beschichtung von ca. 0,6 nm errechnen.

**Beispiel 4:**

Um ein Maß für die Aktivität des unbeschichteten und des mit der ionischen Flüssigkeit beschichteten Nickelkatalysators zu ermitteln, wurden die beiden genannten Katalysatoren in die Umsetzung von Cyclooctadien mit Wasserstoff eingesetzt.

Die Hydrierung von Cyclooctadien mittels des unbeschichteten Nickelkatalysators erfolgte unter den folgenden Reaktionsparametern:

$$d_{kat} = 125\text{-}250 \text{ Mikrometer}$$

$$m^0_{Cyclooctadien} = 0,\ 178 \text{ g}$$

$$V_{Dodecan} = 150 \text{ ml}$$

$$T = 50°C$$

$$p_{H2} = 50 \text{ bar}$$

$$n = 2000 \text{ l/min}$$

$$m_{Kat} = 0{,}1134.$$

Die Hydrierung von Cyclooctadien mittels des mit der ionischen Flüssigkeit beschichteten Nickelkatalysators erfolgte unter den folgenden Reaktionsbedingungen:

$$d_{Kat} = 125\text{-}250 \text{ Mikrometer}$$

$$m^0_{Cyclooctadien} = 0{,}172 \text{ g}$$

$$V_{Dodecan} = 150 \text{ ml}$$

$$T = 50°C$$

$$p_{H2} = 50 \text{ bar}$$

$$n = 2000 \text{ l/min}$$

$$m_{Kat} = 0,8145.$$

In der Fig. 3 sind die Umsätze der Hydrierung von Cyclooctadien für den unbeladenen Nickelkatalysator (Kurve 1c) und den mit der ionischen Flüssigkeit beschichteten Nickelkatalysator (Kurve 2c) in Abhängigkeit von der modifizierten Versuchszeit dargestellt.

Den Kurvenverläufen ist zu entnehmen, dass die notwendige Versuchszeit für einen 50 %igen Umsatz an Cyclooctadien für den mit der ionischen Flüssigkeit beschichteten Nickelkatalysator ca. 5000 kg s $m^3$ beträgt, während für den unbeschichteten Nickelkatalysator für den gleichen Umsatz eine Versuchszeit von ca. 15000 kg s $m^3$ notwendig ist. Daraus folgt, dass die Reaktionsrate für den mit der ionischen Flüssigkeit beschichteten Nickelkatalysator um etwa den Faktor 3 langsamer ist.

Nach der Reaktion wurde der mit der ionischen Flüssigkeit beladene Nickelkatalysator bei einer Temperatur von 110°C im Vakuum getrocknet und anschließend sein Porensystem mittels BET- und Hg-Porosimetrie-Messungen charakterisiert. Die Abhängigkeit des Porenvolumens vom Porendurchmesser und die Abhängigkeit der inneren Oberfläche vom Porendurchmesser ist durch die Kurvenverläufe 3a bzw. 3b der Figuren 1 bzw. 2 dargestellt. Die Abnahme des Porenvolumens bzw. der inneren Oberfläche des beschichteten Nickelkatalysators ist vermutlich darauf zurückzuführen, dass die Poren mit Resten des Lösungsmittels Dodecan belegt sind, das einen Siedepunkt von 216°C hat.

**Beispiel 5 :**

Um die Selektivität des unbeschichteten Nickelkatalysators bezüglich Hydrierungsreaktion zu bestimmen, wurde der unbeladene Nickelkatalysator bei der Umsetzung von Cyclooctadien mit Wasserstoff eingesetzt. Die Hydrierung des Cyclooctadiens erfolgte unter den folgenden Reaktionsbedingungen:

$$m_{kat} = 0,11 \text{ g}$$

$$d_{kat} = 125\text{-}250 \text{ Mikrometer}$$

$$m^0_{Cyclooctadien} = 0,18 \text{ g}$$

$$V_{Dodecan} = 150 \text{ ml}$$

$$T = 50°C$$

$$P_{H2} = 50 \text{ bar}$$

$$n = 2000 \text{ 1/min}$$

$$C^0_{Cyclooctadien} = 10,9 \text{ mol/m}^3$$

$$C^0_{H2} = 194 \text{ mol/m}^3.$$

In der Fig. 4 ist der Verlauf des Restanteils an Cyclooctadien und der Ausbeuten an cis-Cycloocten und Cyclooctan durch die Kurven mit den Bezugszeichen 1d, 1e bzw. 1f dargestellt. Der Kurve 1e kann entnommen werden, dass der Anteil an cis-Cycloocten während der Hydrierungsreaktion einen maximalen Wert von ca. 20 % erreicht, was einer Selektivität bzgl. cis-Cycloocten von 26 % entspricht.

In der Fig. 5 ist mittels der Kurven 1g und 1h der Verlauf der Selektivitäten bzgl. cis-Cycloocten bzw. Cyclooctan der in Rede stehenden Umsetzung dargestellt. Die Kurve 1g zeigt einen verhältnismäßig schnellen Abfall der cis-Cycloocten-Selektivität mit zunehmendem Cyclooctadien-Umsatz.

**Beispiel 6:**

Um die Selektivität des mit der ionischen Flüssigkeit beschichteten Nickelkatalysators bzgl. Hydrierungsreaktionen zu ermitteln, wurde der beschichtete Nickelkatalysator bei der Hydrierung von Cyclooctadien unter den nachstehenden Reaktionsbedingungen eingesetzt:

$$m_{kat} = 0{,}81 \text{ g}$$

$$d_{kat} = 125\text{-}250 \text{ Mikrometer}$$

$$m^0_{cyclooctadien} = 0{,}17 \text{ g}$$

$$V_{Dodecan} = 150 \text{ ml}$$

$$T = 50°C$$

$$p_{H2} = 50 \text{ bar}$$

$$n = 2000 \text{ l/min.}$$

Der Verlauf der Anteile an Cyclooctadien, an cis-Cycloocten und an Cyclooctan über die genannte Hydrierungsreaktion hinweg ist durch die mit den Bezugszeichen 2d, 2e bzw. 2f belegten Kurven dargestellt. Die Kurve 2e weist einen maximalen Anteil an cis-Cycloocten von 65 % auf, was einer Selektivität bezüglich cis-Cycloocten von 72 % entspricht. Der Verlauf der Selektivitäten bzgl. cis-Cycloocten und Cyclooctan während der in Rede stehenden Reaktion ist in der Fig. 7 mittels der mit den Bezugszeichen 2g bzw. 2h belegten Kurven dargestellt. Im Vergleich zum unbeschichteten Nickelkatalysator zeigt der mit der ionischen Flüssigkeit beschichtete erfindungsgemäße Nickelkatalysator bei der Hydrierung von Cyclooctadien einen vergleichsweise langsamen Abfall der cis-Cycloocten-Selektivität mit zunehmendem Cyclooctadien-Umsatz.

## Patentansprüche

1. Poröser heterogener geträgerter Übergangsmetallkatalysator, **dadurch gekennzeichnet, dass** die innere Oberfläche des Katalysators mit einer ionischen Flüssigkeit (IL) beschichtet ist, wobei die Metalle des Übergangsmetallkatalysators ausgewählt sind aus der Gruppe bestehend aus Ni, Co, Cu, Fe, Ru, Rh, Ir, Pd, sowie Pt und dass der poröse heterogene Katalysator selbst ein präformierter Katalysator ist.

2. Katalysator nach Anspruch 1, **dadurch gekennzeichnet, dass** der Katalysator als Pulver oder als Formkörper ausgebildet ist.

3. Katalysator nach einem der voranstehenden Ansprüche, **dadurch gekennzeichnet, dass** die BET-Oberfläche des Katalysators ohne die IL-Beschichtung 10 bis 300 $m^2/g$ beträgt, vorzugsweise 15 bis 80 $m^2/g$, besonders bevorzugt 20 bis 50 $m^2/g$.

4. Katalysator nach einem der voranstehenden Ansprüche, **dadurch gekennzeichnet, dass** das integrale Porenvolumen des Katalysators ohne die IL-Beschichtung größer als 100 $mm^3/g$ ist, vorzugsweise größer als 180 $mm^3/g$.

5. Katalysator nach einem der voranstehenden Ansprüche, **dadurch gekennzeichnet, dass** das Kation der ionischen Flüssigkeit ausgewählt ist aus Verbindungen der nachstehenden allgemeinen Formeln IL-1 bis IL-23:

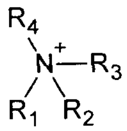

(IL-1)

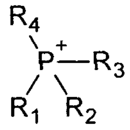

(IL-2)

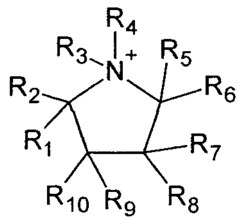

(IL-3)

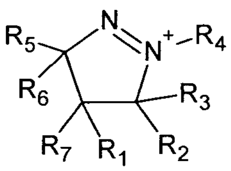

(IL-4)

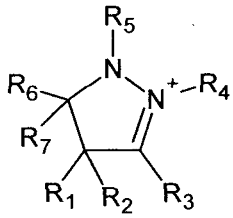

(IL-5)

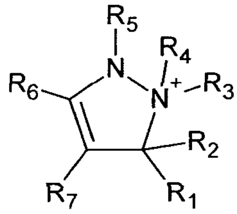

(IL-6)

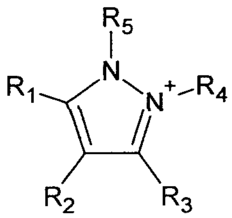

(IL-7)

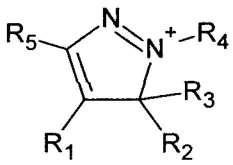

(IL-8)

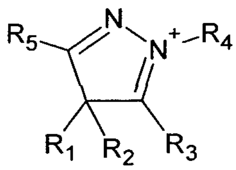

(IL-9)

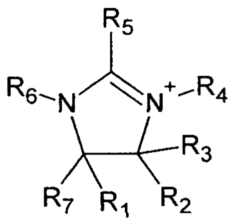

(IL-10)

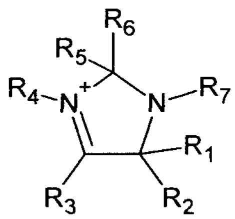

(IL-11)

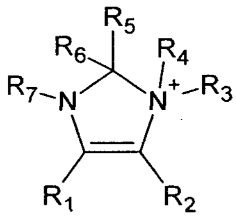

(IL-12)

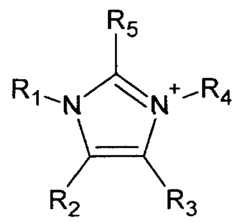

(IL-13)

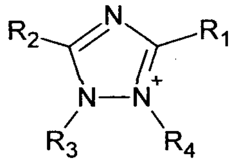

(IL-14)

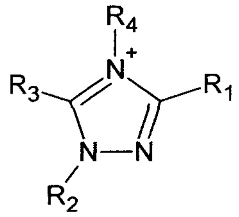

(IL-15)

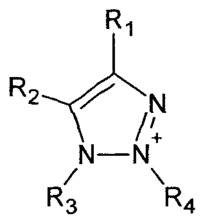

(IL-16)

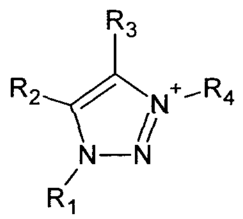

(IL-17)

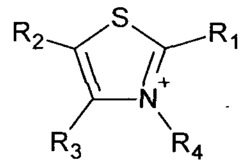

(IL-18)

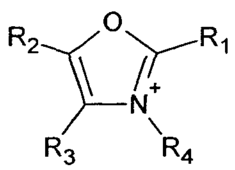

(IL-19)

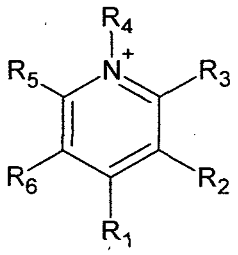

(IL-20)

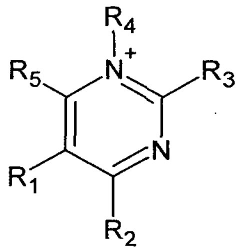

(IL-21)

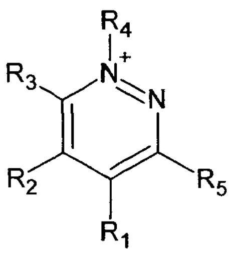

(IL-22)

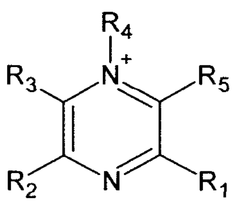

(IL-23)

worin die Reste $R_1$, $R_2$, $R_3$, $R_4$, $R_5$, $R_6$, $R_7$, $R_8$, $R_9$ und $R_{10}$ jeweils unabhängig voneinander Reste sein können ausgewählt aus der Gruppe bestehend aus Wasserstoff, funktionelle Gruppen, Aryl, Alkyl, Aryloxy, Alkyloxy, Halogen, Heteroatom und/oder Heterocyclus substituiertes $C_1$-$C_{18}$-Alkyl, durch ein oder mehrere nicht benachbarte Sauerstoffatome und/oder Schwefelatome und/oder ein oder mehrere substituierte oder unsubstituierte Iminogruppen unterbrochenes $C_2$-$C_{18}$-Alkyl, $C_6$-$C_{12}$-Aryl, $C_5$-$C_{12}$-Cycloalkyl, ein fünf- bis sechsgliedriger Sauerstoff-, Stickstoff- und/oder Schwefelatome aufweisender Heterocyclus,
wobei zwei der genannten Reste miteinander unter Ausbildung eines ungesättigten oder gesättigten Ringsegments verknüpft sein können, das gegebenenfalls durch ein oder mehrere Sauerstoff- und/oder Schwefelatome und/oder ein oder mehrere substituierte oder unsubstituierte Iminogruppen unterbrochen sein kann, wobei das Ringsegment durch funktionelle Gruppen, Aryl-, Alkyl-, Aryloxy-, Alkyloxy-, Halogen-, Heteroatom- und/oder Heterocyclus-Reste substituiert sein kann,
und wobei $R_4$ darüber hinaus ausgewählt sein kann aus der Gruppe der Reste bestehend aus $C_1$-$C_{18}$-Alkyloyl,

$C_1$-$C_{18}$-Alkyloxycarbonyl, $C_5$-$C_{12}$-Cycloalkylcarbonyl und $C_6$-$C_{12}$-Aryloyl, wobei die Mitglieder der genannten Gruppe jeweils durch einen oder mehrere funktionelle Gruppen, Aryl-, Alkyl-, Aryloxy-, Alkyloxy-, Halogen-, Heteroatome und/oder Heterocyclus-Reste substituiert sein können, wobei sich die Angaben $C_1$-$C_{18}$, $C_5$-$C_{12}$, bzw. $C_6$-$C_{12}$ auf die Alkylkette beziehen.

**6.** Katalysator nach einem der voranstehenden Ansprüche, **dadurch gekennzeichnet, dass** das Anion der ionischen Flüssigkeit ausgewählt ist aus der Gruppe bestehend aus $F^-$, $Cl^-$, $Br^-$, $I^-$, $PF_6^-$, $BF_4^-$, Alkylsulfat, vorzugsweise ein $C_1$ bis $C_{18}$ Alkylsulfat, Ethersulfat, Acetat, Trifluoracetat, Triflat, Sulfat, Hydrogensulfit, Methylsulfat, Ethylsulfat, Sulfit, Hydrogensulfit, Aluminiumchloride, vorzugsweise $AlCl_4^-$, $Al_2Cl_7^-$ oder $Al_3Cl_{10}^-$, Aluminiumtribromid, Nitrit, Nitrat, Metallkomplexe, beispielsweise Metallhalogenide wie Kupferchlorid $CuCl_2^-$, Phosphate, Phosphat, Hydrogenphosphat, Dihydrogenphosphat, Carbonat, Hydrogencarbonat, Sulfonat, Tosylat, Bis(trifluormethylsulfonyl)imid, Cyanid und Isocyanat.

**7.** Katalysator nach einem der voranstehenden Ansprüche, **dadurch gekennzeichnet, dass** in der IL-Beschichtung ein homogener Katalysator enthalten ist.

**8.** Katalysator nach einem der voranstehenden Ansprüche, **dadurch gekennzeichnet, dass** der poröse heterogene Katalysator ein geträgerter Katalysator ist, wobei der Katalysatorträger hergestellt ist unter Verwendung eines Materials ausgewählt aus der Gruppe bestehend aus Titanoxid, Siliciumoxid, Aluminiumoxid, Zirkoniumoxid, Magnesiumoxid, Siliciumcarbid, Magnesiumsilicat, Zinkoxid, Zeoliten und Nanomaterialien, wie beispielsweise Kohlenstoffnanotubes oder Kohlenstoffnanofasern.

**9.** Verfahren zur Herstellung eines porösen heterogenen Übergangsmetallkatalysators, dessen innere Oberfläche mit einer ionischen Flüssigkeit beschichtet ist, umfassend die folgenden Schritte:

a) das Lösen einer ionischen Flüssigkeit in einem geeigneten Lösemittel;
b) das Inkontaktbringen eines porösen heterogenen geträgerten Übergangsmetallkatalysators mit dem die ionische Flüssigkeit enthaltenden Lösemittel gemäß Schritt a);
c) das Entfernen des Lösemittels,

wobei die Metalle des Übergangsmetallkatalysators ausgewählt sind aus der Gruppe bestehend aus Ni, Co, Cu, Fe, Ru, Rh, Ir, Pd, sowie Pt und wobei der poröse heterogene Katalysator selbst ein präformierter Katalysator ist.

**10.** Verfahren nach Anspruch 9, **dadurch gekennzeichnet, dass** der poröse heterogene Katalysator ein Katalysator nach einem der Ansprüche 1 bis 8 ist.

**11.** Verfahren nach Anspruch 9, wobei das Inkontaktbringen des porösen heterogenen geträgerten Übergangsmetallkatalysators mit dem die ionische Flüssigkeit enthaltenden Lösemittel erfolgt durch:

a) das Einbringen eines porösen heterogenen geträgerten Übergangsmetallkatalysators in eine Kammer, in der eine durch eine Besaugung der Kammer bewirkte Strömung sowie ein Unterdruck herrscht;
b) das Einleiten des die ionische Flüssigkeit enthaltenden Lösemittels in die Kammer;
c) das Bilden eines Gemisches aus Gas und dem die ionische Flüssigkeit enthaltenden Lösemittel;
d) das kontinuierliche Durchführen des Gemisches aus Schritt c) durch den Katalysator;

**12.** Verwendung eines Katalysators nach einem der Ansprüche 1 bis 8 zur selektiven Hydrierung von ungesättigten Gruppen mehrfach ungesättigter Verbindungen, insbesondere zur Hydrierung von aromatischen Verbindungen zu Cycloolefinen, zur Hydrierung von Acetylen zu Ethylen, zur Hydrierung von Diolefinen in Monolefine, insbesondere zur Hydrierung von Butadien zu 1-Buten und/oder 2-Buten, zur Hydrierung von Phenylacetylen zu Styrol, zur Hydrierung von Methylacetylen und Propadien zu Propen, zur Hydrierung von Olefinen und Diolefinen in aromatischen Strömen ohne Verlust an Aromaten oder zur Hydrierung von Cyclododecatrien zu Cyclododecen.

## Claims

**1.** Porous heterogeneous supported transition metal catalyst, **characterized in that** the inner surface of the catalyst is coated with an ionic liquid (IL), wherein the metals of the transition metal catalyst are selected from the group consisting of Ni, Co, Cu, Fe, Ru, Rh, Ir, Pd and Pt, and that the porous heterogeneous catalyst itself is a preformed

catalyst.

2. Catalyst according to claim 1, **characterized in that** the catalyst is formed as powder or as a shaped body.

3. Catalyst according to one of the previous claims, **characterized in that** the BET surface area of the catalyst without the IL-coating is 10 to 300 $m^2/g$, preferably 15 to 80 $m^2/g$, particularly preferably 20 to 50 $m^2/g$.

4. Catalyst according to one of the previous claims, **characterized in that** the integral pore volume of the catalyst without the IL-coating is greater than 100 $mm^3/g$, preferably greater than 180 $mm^3/g$.

5. Catalyst according to one of the previous claims, **characterized in that** the cation of the ionic liquid is selected from compounds of the following general Formulae IL-1 to IL-23:

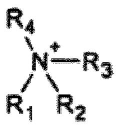

(IL-1)

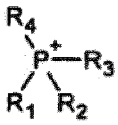

(IL-2)

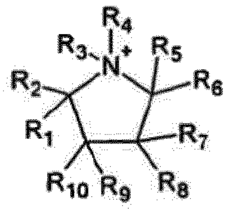

(IL-3)

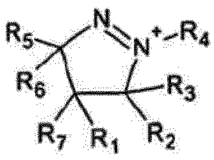

(IL-4)

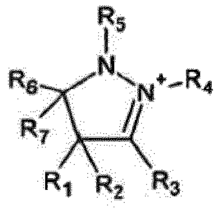

(IL-5)

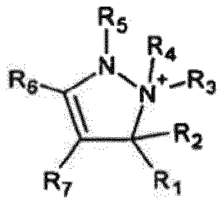

(IL-6)

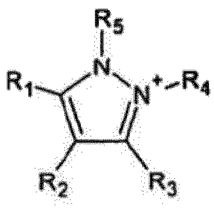

(IL-7)

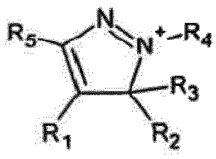

(IL-8)

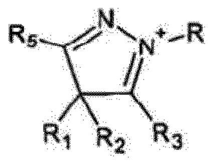

(IL-9)

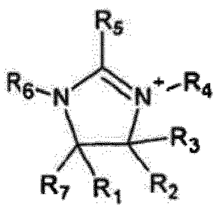

(IL-10)

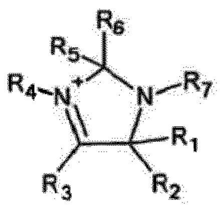

(IL-11)

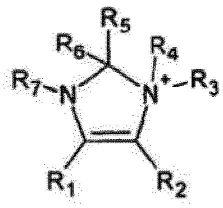

(IL-12)

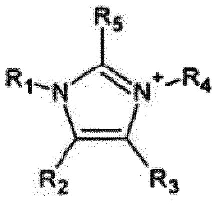

(IL-13)

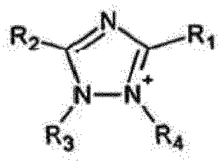

(IL-14)

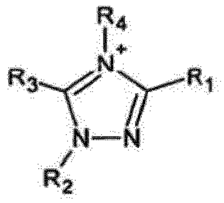

(IL-15)

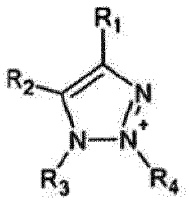

(IL-16)

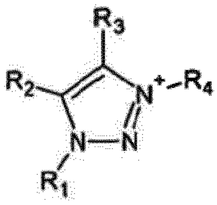

(IL-17)

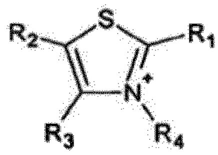

(IL-18)

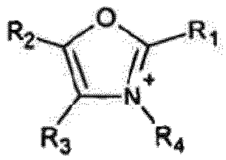

(IL-19)

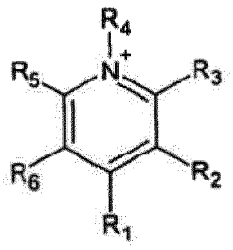

(IL-20)

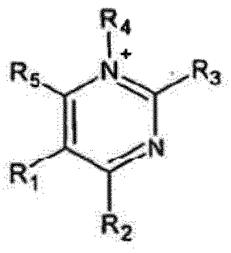

(IL-21)

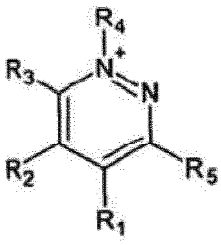

(IL-22)

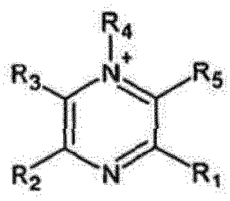

(IL-23)

in which the radicals $R_1$, $R_2$, $R_3$, $R_4$, $R_5$, $R_6$, $R_7$, $R_8$, $R_9$ and $R_{10}$ each independently of one another can be radicals selected from the group consisting of hydrogen, functional groups, aryl, alkyl, aryloxy, alkyloxy, halogen, heteroatom and/or heterocycle-substituted $C_1$-$C_{18}$ alkyl, $C_2$-$C_{18}$ alkyl; $C_6$-$C_{12}$ aryl, $C_5$-$C_{12}$ cycloalkyl interrupted by one or more non-adjacent oxygen atoms and/or sulphur atoms and/or one or more substituted or unsubstituted imino groups, a five- to six-membered heterocycle having oxygen, nitrogen and/or sulphur atoms,
wherein two of the named radicals can be linked together with formation of an unsaturated or saturated ring segment which can optionally be interrupted by one or more oxygen and/or sulphur atoms and/or one or more substituted or unsubstituted imino groups, wherein the ring segment can be substituted by functional groups, aryl, alkyl, aryloxy, alkyloxy, halogen, heteroatom and/or heterocycle radicals,
and wherein $R_4$ in addition can be selected from the group of radicals consisting of $C_1$-$C_{18}$ alkyloyl, $C_1$-$C_{18}$ alkyloxycarbonyl, $C_5$-$C_{12}$ cycloalkylcarbonyl and $C_6$-$C_{12}$ aryloyl, wherein the members of the named group can each be substituted by one or more functional groups, aryl, alkyl, aryloxy, alkyloxy, halogen, heteroatoms and/or heterocycle radicals, wherein $C_1$-$C_{18}$, $C_5$-$C_{12}$, or $C_6$-$C_{12}$ refer to the alkyl chain.

**6.** Catalyst according to one of the previous claims, **characterized in that** the anion of the ionic fluid is selected from

the group consisting of $F^-$, $Cl^-$, $Br^-$, $I^-$, $PF_6^-$, $BF_4^-$, alkyl sulphate, preferably a $C_1$ to $C_{18}$ alkyl sulphate, ether sulphate, acetate, trifluoroacetate, triflate, sulphate, hydrogensulphite, methyl sulphate, ethyl sulphate, sulphite, hydrogensulphite, aluminium chlorides, preferably $AlCl_4^-$, $Al_2Cl_7^-$ or $Al_3Cl_{10}^-$, aluminium tribromide, nitrite, nitrate, metal complexes, for example metal halides such as copper chloride $CuCl_2^-$, phosphates, phosphate, hydrogen phosphate, dihydrogen phosphate, carbonate, hydrogen carbonate, sulphonate, tosylate, bis(trifluoromethylsulphonyl)imide, cyanide and isocyanate.

**7.** Catalyst according to one of the previous claims, **characterized in that** the IL-coating contains a homogeneous catalyst.

**8.** Catalyst according to one of the previous claims, **characterized in that** the porous heterogeneous catalyst is a supported catalyst, wherein the catalyst support is produced using a material selected from the group consisting of titanium oxide, silicon oxide, aluminium oxide, zirconium oxide, magnesium oxide, silicon carbide, magnesium silicate, zinc oxide, zeolites and nanomaterials, such as for example carbon nanotubes or carbon nanofibres.

**9.** Process for the preparation of a porous heterogeneous transition metal catalyst, the inner surface of which is coated with an ionic liquid, comprising the following steps:

a) the dissolving of an ionic liquid in a suitable solvent;
b) the bringing of a porous heterogeneous supported transition metal catalyst into contact with the solvent containing the ionic liquid according to step a);
c) the removal of the solvent,

wherein the metals of the transition metal catalyst are selected from the group consisting of Ni, Co, Cu, Fe, Ru, Rh, Ir, Pd and Pt, and wherein the porous heterogeneous catalyst itself is a preformed catalyst.

**10.** Process according to claim 9, **characterized in that** the porous heterogeneous catalyst is a catalyst according to one of claims 1 to 8.

**11.** Process according to claim 9, wherein the porous heterogeneous supported transition metal catalyst is brought into contact with the solvent containing the ionic liquid by:

a) the introduction of a porous heterogeneous supported transition metal catalyst into a chamber, in which a flow brought about by the application of suction to the chamber and a below-atmospheric pressure prevails;
b) the introduction into the chamber of the solvent containing the ionic liquid;
c) the formation of a mixture of gas and the solvent containing the ionic liquid;
d) the continuous passing of the mixture from step c) through the catalyst.

**12.** Use of a catalyst according to one of claims 1 to 8 for the selective hydrogenation of unsaturated groups of polyunsaturated compounds, in particular for the hydrogenation of aromatic compounds to form cycloolefins, for the hydrogenation of acetylene to form ethylene, for the hydrogenation of diolefins into monolefins, in particular for the hydrogenation of butadiene to form 1-butene and/or 2-butene, for the hydrogenation of phenylacetylene to form styrene, for the hydrogenation of methylacetylene and propadiene to form propene, for the hydrogenation of olefins and diolefins in aromatic flows without loss of aromatics or for the hydrogenation of cyclododecatriene to form cyclododecene.

**Revendications**

**1.** Catalyseur à métal de transition poreux,hétérogène et sur support, **caractérisé en ce que** la surface intérieure dudit catalyseur est revêtue d'un liquide ionique (IL), dans lequel les métaux du catalyseur de métal de transition sont choisis dans le groupe consistant en Ni, Co, Cu, Fe, Ru, Rh, Ir, Pd et Pt et **en ce que** le catalyseur hétérogène poreux est lui-même un catalyseur préformé.

**2.** Catalyseur selon la revendication 1, **caractérisé en ce que** ledit catalyseur est réalisé sous forme de poudre ou de corps moulé.

**3.** Catalyseur selon l'une des revendications précédentes, **caractérisé en ce que** la surface BET dudit catalyseur

sans ledit revêtement IL est de 10 à 300 $m^2/g$, de préférence de 15 à 80 $m^2/g$, avec une préférence particulière de 20 à 50 $m^2/g$.

**4.** Catalyseur selon l'une des revendications précédentes, **caractérisé en ce que** le volume poreux intégral dudit catalyseur sans ledit revêtement IL est supérieur à 100 $mm^3/g$, de préférence supérieur à 180 $mm^3/g$.

**5.** Catalyseur selon l'une des revendications précédentes, **caractérisé en ce que** le cation dudit liquide ionique est choisi parmi les composés répondant aux formules générales IL-1 à Il-23 ci-après :

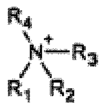

(IL-1)

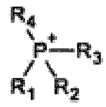

(IL-2)

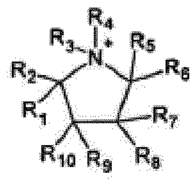

(IL-3)

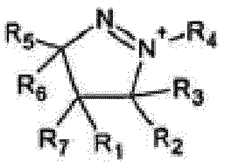

(IL-4)

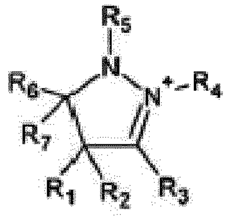

(IL-5)

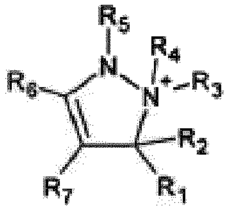

(IL-6)

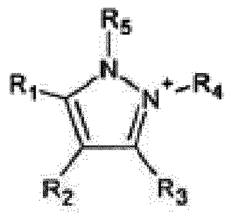

(IL-7)

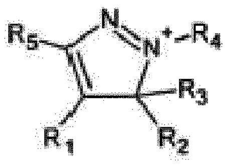

(IL-8)

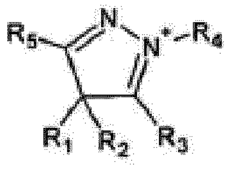

(IL-9)

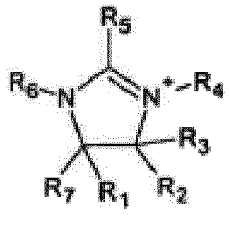

(IL-10)

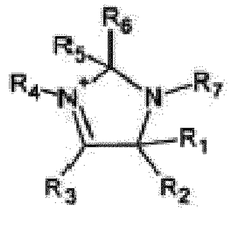

(IL-11)

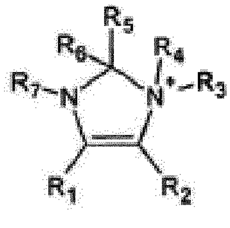

(IL-12)

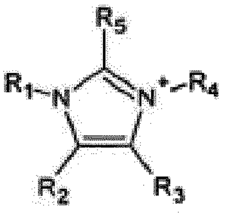

(IL-13)

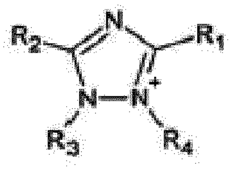

(IL-14)

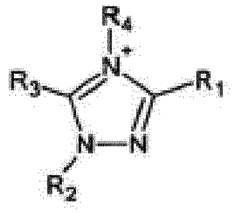

(IL-15)

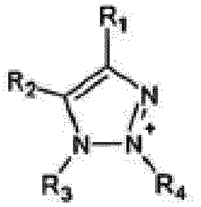

(IL-16)

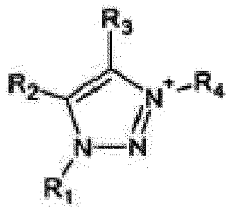

(IL-17)

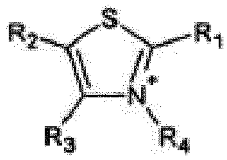

(IL-18)

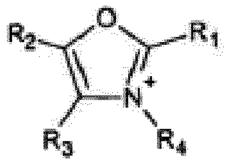

(IL-19)

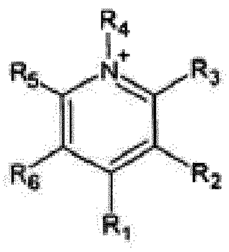

(IL-20)

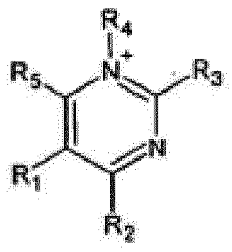

(IL-21)

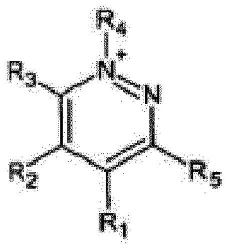

(IL-22)

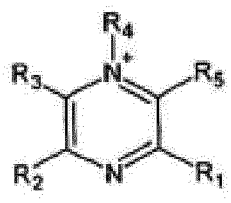

(IL-23)

dans lesquelles les radicaux $R_1$, $R_2$, $R_3$, $R_4$, $R_5$, $R_6$, $R_7$, $R_8$, $R_9$ et $R_{10}$ peuvent, indépendamment l'un de l'autre, être des radicaux choisis dans le groupe constitué d'hydrogène, de groupements fonctionnels, d'aryle, d'alkyle, d'aryloxy, d'alkyloxy, d'halogène, d'alkyle en $C_1$-$C_{18}$ substitué avec un hétéroatome et/ou un hétérocycle, d'alkyle en $C_2$-$C_{18}$, aryle en $C_6$-$C_{12}$, cycloalkyl en $C_5$-$C_{12}$ interrompus par un ou plusieurs atomes d'oxygène et ou atomes de soufre non-contigus et/ou un ou plusieurs groupements imine substitués ou non-substitués, d'un hétérocycle penta- ou hexagonale comportant des atomes d'oxygène, d'azote et/ou de soufre,
deux des radicaux mentionnés pouvant être liés l'un à l'autre en formant un segment d'anneau insaturé ou saturé, lequel peut, le cas échéant, être interrompu par un ou plusieurs atomes d'oxygène et/ou de soufre et/ou par un ou plusieurs groupements imine substitués ou non-substitués, ledit segment d'anneau pouvant être substitué avec des groupements fonctionnels, des radicaux aryle, alkyle, aryloxy, alkyloxy, d'halogène, d'hétéroatome et/ou d'hétérocycle.
et $R_4$ pouvant, en outre, être choisi dans le groupe de radicaux constitué d'alkyloyle en $C_1$-$C_{18}$, d'alkyloxycarbonyle en $C_1$-$C_{18}$, de cycloalkylcarbonyle en $C_5$-$C_{12}$ et d'aryloyle en $C_6$-$C_{12}$, les représentants dudit groupe pouvant chacun être substitués avec un ou plusieurs groupements fonctionnels, des radicaux aryle, alkyle, aryloxy, alkyloxy, d'halogène, d'hétéroatome et/ou d'hétérocycle, les indications $C_1$-$C_{18}$, $C_5$-$C_{12}$ et, respective-

ment, $C_6$-$C_{12}$ se rapportant à la chaîne alkyle.

**6.** Catalyseur selon l'une des revendications précédentes, **caractérisé en ce que** l'anion dudit liquide ionique est choisi dans le groupe constitué de $F^-$, $Cl^-$ $Br^-$, $I^-$, $PF_6^-$, $BF_4^-$, de sulfate d'alkyle, préférentiellement de sulfate d'alkyle en $C_1$ à $C_{18}$, de sulfate d'éther, d'acétate, de trifluoroacétate, de triflate, de sulfate, de bisulfite, de sulfate de méthyle, de sulfate d'éthyle, de sulfite, de bisulfite, de chlorures d'aluminium, préférentiellement de $AlCl_4^-$, $Al_2Cl_7^-$ ou $Al_3Cl_{10}^-$ , de tribromure d'aluminium, de nitrite, de nitrate, de complexes de métaux, par exemple d'halogénures de métaux comme le chlorure de cuivre $CuCl_2^-$, de phosphates, de phosphate, d'hydrogénophosphate, de dihydrogénophosphate, de carbonate, de bicarbonate, de sulfonate, de tosylate, de bis-(trifluorométhylsulfonyl)imide, de cyanure et d'isocyanate.

**7.** Catalyseur selon l'une des revendications précédentes, **caractérisé en ce qu'**un catalyseur homogène est contenu dans ledit revêtement IL.

**8.** Catalyseur selon l'une des revendications précédentes, **caractérisé en ce que** ledit catalyseur poreux hétérogène est un catalyseur sur support, le support de catalyseur étant préparé en utilisant un matériau choisi dans le groupe constitué d'oxyde de titane, d'oxyde de silicium, d'oxyde d'aluminium, d'oxyde de zirconium, d'oxyde de magnésium, de carbure de silicium, de silicate de magnésium, d'oxyde de zinc, de zéolithes et de nanomatériaux comme, par exemple, les nanotubes de carbone ou les nanofibres de carbone.

**9.** Procédé de préparation catalyseur à métal de transition poreux hétérogène dont la surface intérieure est revêtue d'un liquide ionique, comprenant les étapes suivantes :

a) dissolution d'un liquide ionique dans un solvant approprié;
b) mise en contact d'un catalyseur à métaux de transition de nature poreuse, hétérogène et sur support avec le solvant selon l'étape à) contenant ledit liquide ionique;
c) élimination du solvant,

les métaux du catalyseur à métal de transition étant choisis dans le groupe constitué par Ni, Co, Cu, Fe, Ru, Rh, Ir, Pd et Pt et le catalyseur hétérogène poreux étant lui-même un catalyseur préformé.

**10.** Procédé selon la revendication 10, **caractérisé en ce que** ledit catalyseur poreux hétérogène est un catalyseur selon l'une des revendications 1 à 9.

**11.** Procédé selon la revendication 10, ladite mise en contact du catalyseur à métal de transition poreux hétérogène et sur support avec le solvant contenant ledit liquide ionique étant réalisée par :

a) l'introduction d'un catalyseur de métal de transition poreux hétérogène et sur support dans une chambre, dans laquelle règne un flux provoqué en soumettant ladite chambre à une aspiration ainsi qu'une pression négative;
b) l'introduction du solvant contenant le liquide ionique dans ladite chambre;
c) la formation d'un mélange de gaz et dudit solvant contenant le liquide ionique;
d) le passage en continue du mélange de l'étape c) à travers ledit catalyseur.

**12.** Utilisation d'un catalyseur selon l'une des revendications 1 à 9 pour l'hydrogénation sélective de groupements insaturés de composés polyinsaturés, notamment pour l'hydrogénation de composés aromatiques en cyclooléfines, pour l'hydrogénation d'acétylène en éthylène, pour l'hydrogénation de dioléfines en monooléfines, notamment pour l'hydrogénation de butadiène en butène-1 et/ou butène-2, pour l'hydrogénation de phénacétylène en styrène, pour l'hydrogénation de méthylacétylène et propadiène en propène, pour l'hydrogénation d'oléfines et de dioléfines dans des flux aromatiques sans perte en composés aromatiques ou pour l'hydrogénation de cyclododécatriène en cyclodocécène.

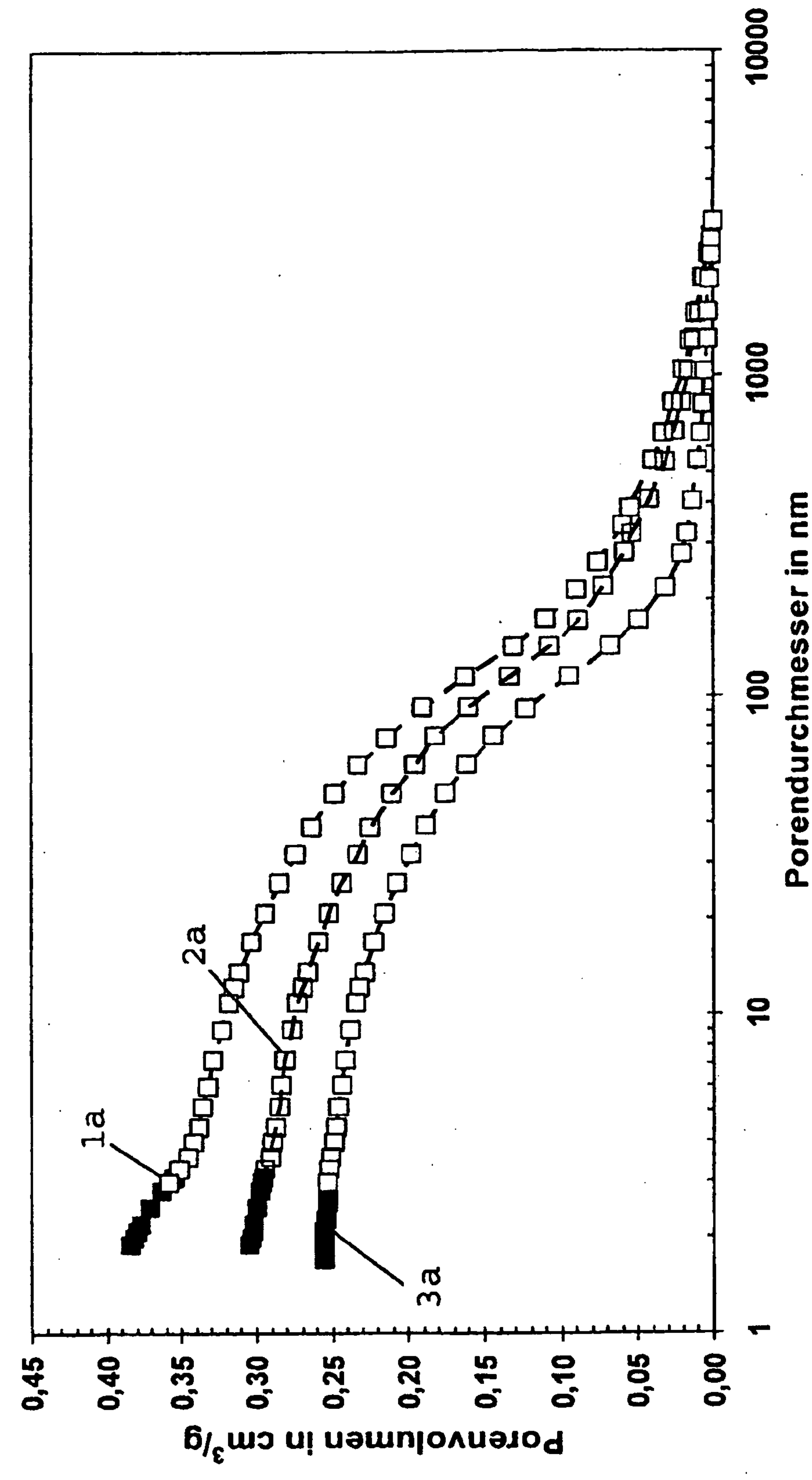

Porenvolumen in cm³/g
0,45
0,40
0,35
0,30
0,25
0,20
0,15
0,10
0,05
0,00
Porendurchmesser in nm
1
10
100
1000
10000
1a
2a
3a


Figur 1

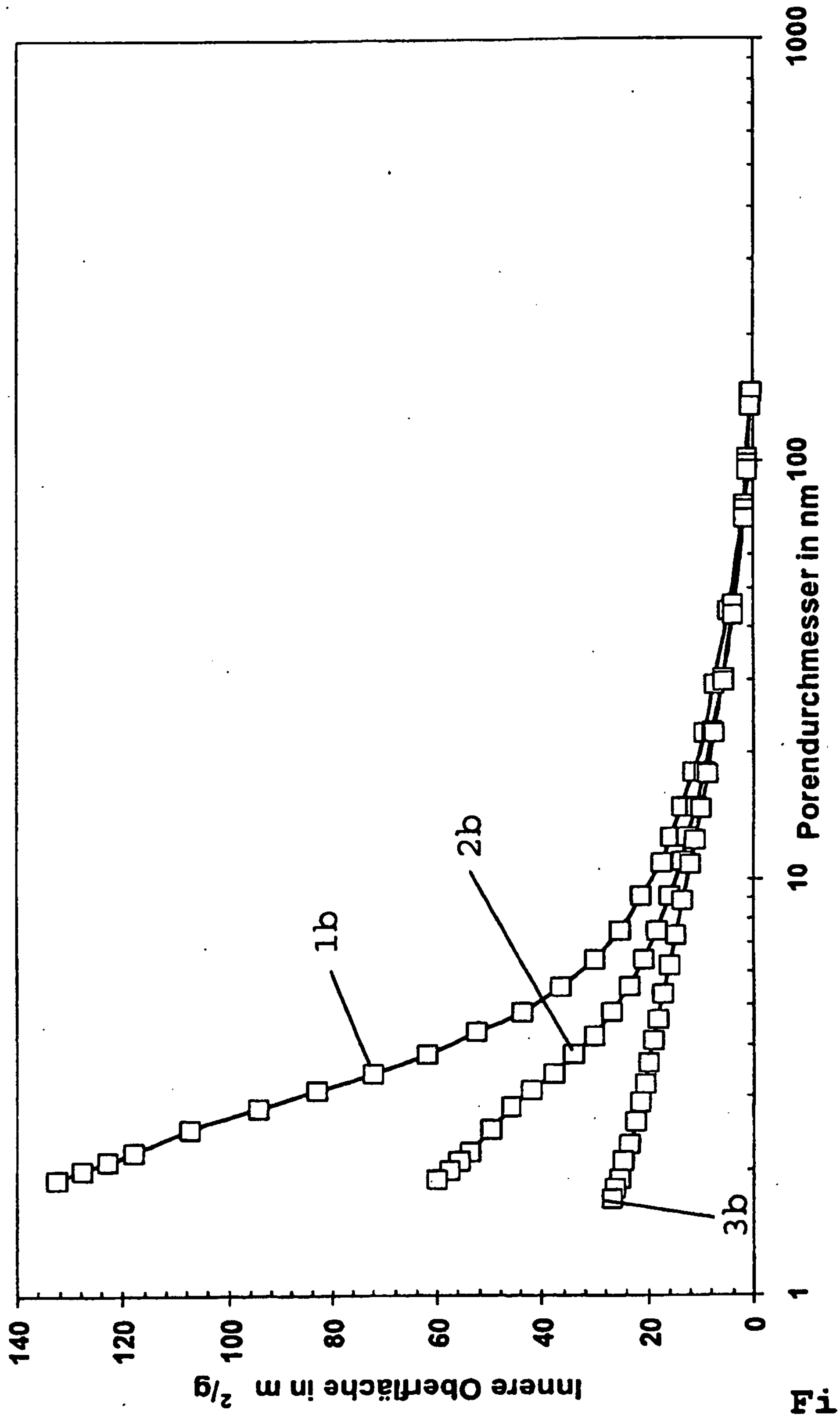
Innere Oberfläche in m 2/g
Porendurchmesser in nm
0
20
40
60
80
100
120
140
1
10
100
1000
1b
2b
3b

Figur 2

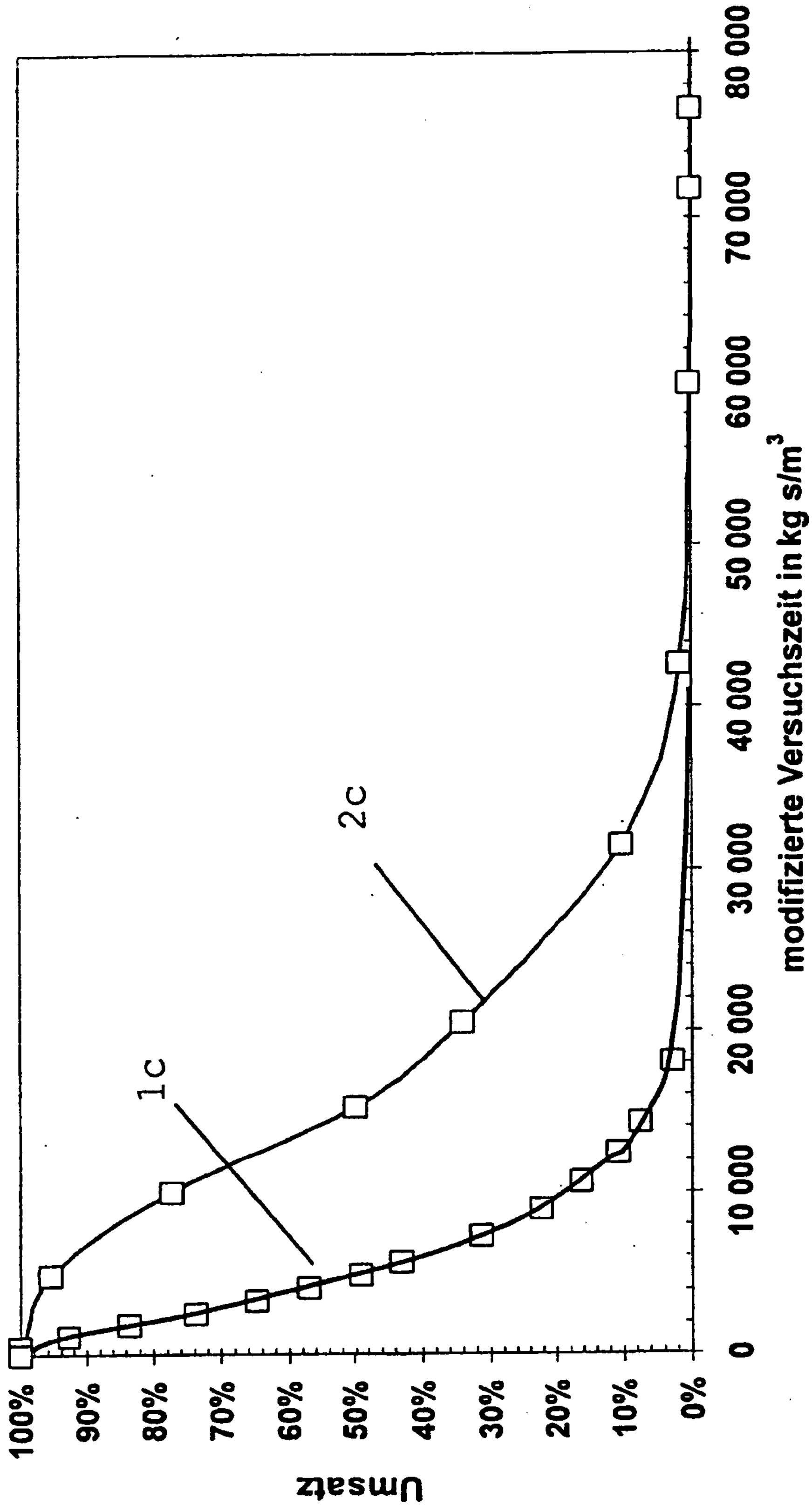
Umsatz
100%
90%
80%
70%
60%
50%
40%
30%
20%
10%
0%
0
10 000
20 000
30 000
40 000
50 000
60 000
70 000
80 000
modifizierte Versuchszeit in kg s/m³
1c
2c

Figur 3

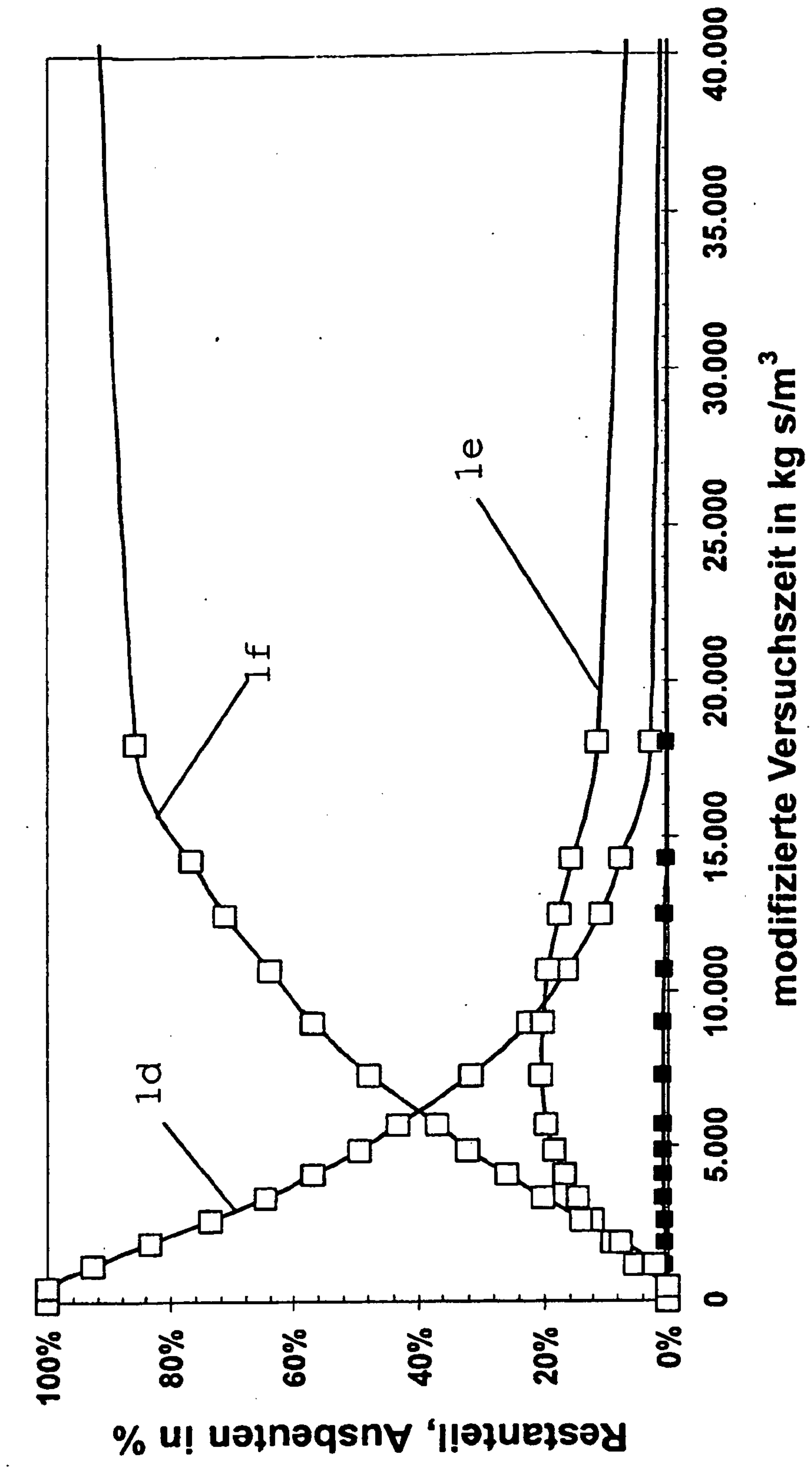
Restanteil, Ausbeuten in %
100%
80%
60%
40%
20%
0%
modifizierte Versuchszeit in kg s/m³
0
5.000
10.000
15.000
20.000
25.000
30.000
35.000
40.000
1d
1e
1f

Figur 4

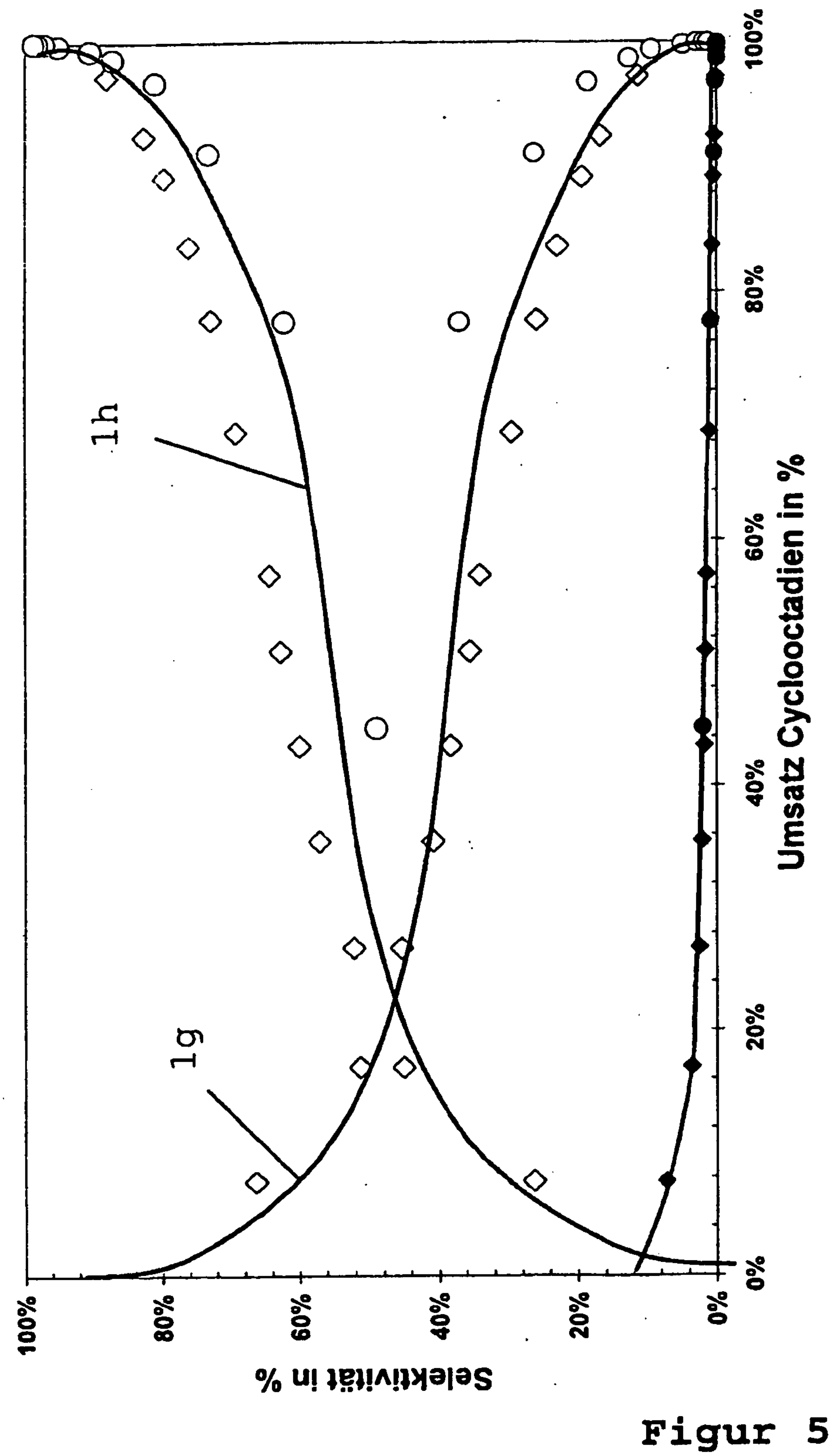
1h
1g
Umsatz Cyclooctadien in %
0%
20%
40%
60%
80%
100%
Selektivität in %
0%
20%
40%
60%
80%
100%

Figur 5

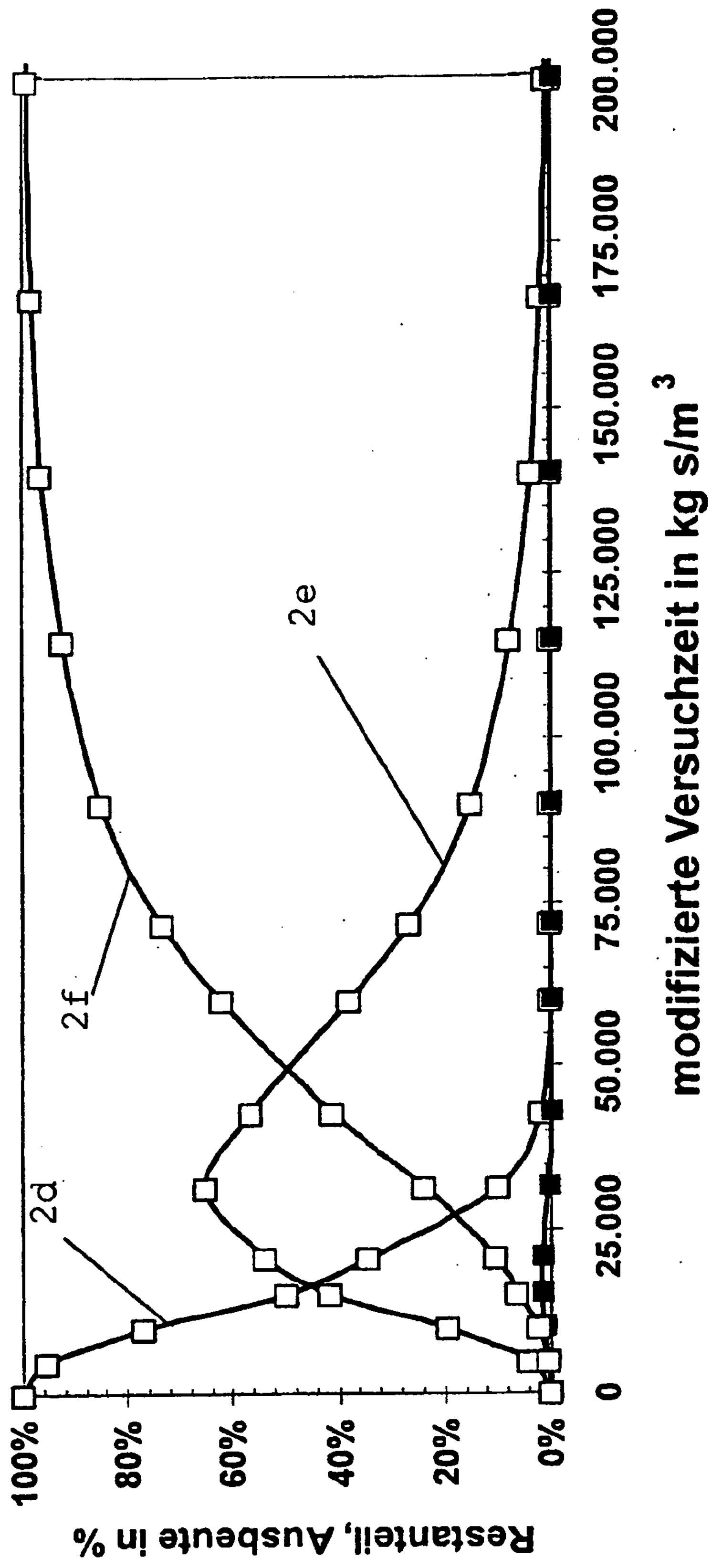
Restanteil, Ausbeute in %
100%
80%
60%
40%
20%
0%
0
25.000
50.000
75.000
100.000
125.000
150.000
175.000
200.000
modifizierte Versuchzeit in kg s/m3
2d
2e
2f

Figur 6

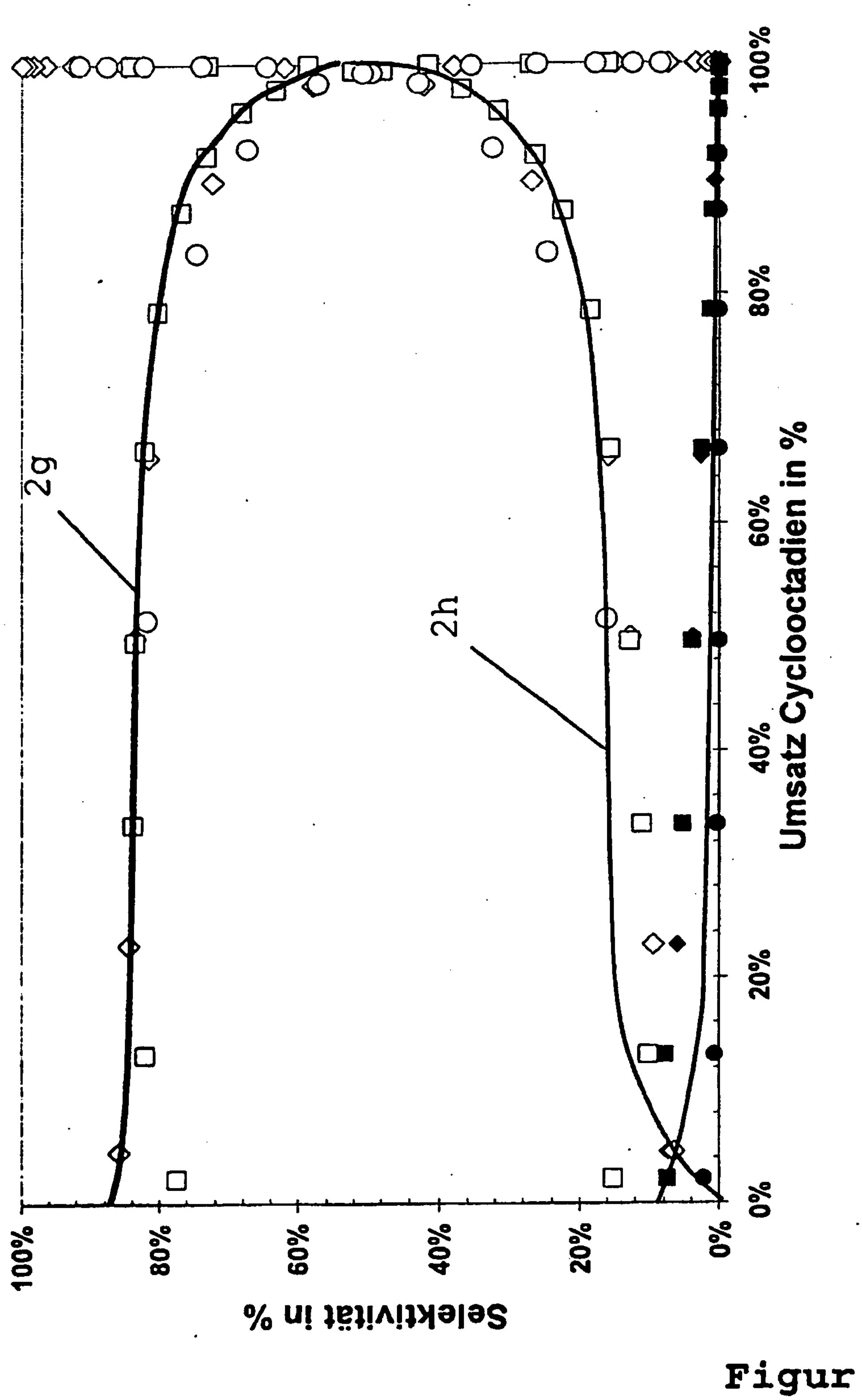
Selektivität in %
Umsatz Cyclooctadien in %
100%
80%
60%
40%
20%
0%
2g
2h

Figur 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- *Green Chem,* 2006, vol. 8, 197-205
- Handbook of Chemistry and Physics. 1999
- *Angewandte Chemie,* 2000, vol. 112, 3926-3945